(19) Europäisches Patentamt
European Patent Office
Office européen des brevets

(11) EP 0 443 918 B1

(12) **FASCICULE DE BREVET EUROPEEN**

(45) Date de publication et mention de la délivrance du brevet:
**26.06.1996 Bulletin 1996/26**

(51) Int Cl.6: **C07D 461/00**, A61K 31/395

(21) Numéro de dépôt: **91400382.7**

(22) Date de dépôt: **14.02.1991**

(54) **Nouveaux dérivés de la 20,21-dinoréburnaménine, leur procédé de préparation et les nouveaux intermédiaires ainsi obtenus, leur application comme médicaments et les compositions les renfermant**

20-21-Dinoreburnamenin-Derivate, Verfahren zu ihrer Herstellung und Zwischenverbindungen, ihre Anwendung als Arzneimittel und diese enthaltende Zusammenstellungen

Derivatives of 20,21-dinoreburnamenine, process for their preparation and intermediates, their use as medicines and compositions containing them

(84) Etats contractants désignés:
**AT BE CH DE DK ES FR GB GR IT LI LU NL SE**

(30) Priorité: **15.02.1990 FR 9001823**

(43) Date de publication de la demande:
**28.08.1991 Bulletin 1991/35**

(73) Titulaire: **ROUSSEL UCLAF**
**F-93230 Romainville (FR)**

(72) Inventeurs:
- **Clemence, François**
  **F-75009 Paris (FR)**
- **Haesslein, Jean-Luc**
  **F-77181 Courtry (FR)**
- **Oberlander, Claude**
  **F-75018 Paris (FR)**

(74) Mandataire: **Vieillefosse, Jean-Claude et al**
**ROUSSEL UCLAF,**
**Département des Brevets,**
**102, Route de Noisy**
**93235 Romainville Cédex (FR)**

(56) Documents cités:
**EP-A- 0 317 427**

Remarques:
Le dossier contient des informations techniques présentées postérieurement au dépôt de la demande et ne figurant pas dans le présent fascicule.

EP 0 443 918 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux dérivés de 20,21-dinoréburnaménine, leur procédé de préparation et les nouveaux intermédiaires ainsi obtenus, leur application comme médicaments et les compositions les renfermant.

Le brevet européen EP 0.317.427 décrit des dérivés de 20,21-dinoréburnaménine comportant sur le cycle A des substituants différents de ceux des produits de la présente demande.

La présente invention a pour objet les produits de formule (I) :

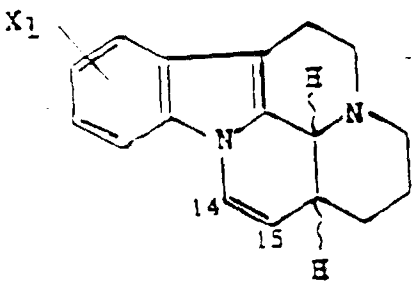

(I)

dans laquelle $X_1$ est choisi parmi les radicaux suivants :

- un radical alkényle linéaire ou ramifié renfermant au plus 18 atomes de carbone ou hydroxyalkyle ayant au plus 8 atomes de carbone,
- un radical cyano, formyle ou acyle ayant de 2 à 4 atomes de carbone,
- un radical alcoxycarbonyle ayant de 2 à 4 atomes de carbone ou carbamoyle,
- un radical phényle,

lesdits produits de formule (I) étant sous toutes les formes isomères possibles racémiques, énantiomères et diastéréoisomères, ainsi que les sels d'addition avec les acides minéraux et organiques desdits produits de formule (I). On préfère les produits dans lesquels $X_1$ est situé en position 9 ou 11 du cycle phényle.

Dans les produits de formule (I), l'atome d'hydrogène en position 3 et l'atome d'hydrogène en position 16 peuvent chacun occuper l'une ou l'autre des orientations alpha et béta, ce qui détermine l'existence de diastéréoisomères cis et trans.

Dans les produits de formule (I) et dans ce qui suit :

- le terme atome d'halogène désigne de préférence l'atome de chlore, mais peut aussi représenter un atome de fluor, de brome ou d'iode,
- le terme radical alkyle linéaire ou ramifié désigne de préférence un radical alkyle inférieur tel que, par exemple, les radicaux méthyle, éthyle, propyle ou isopropyle, mais peut également représenter un radical butyle, isobutyle, sec-butyle, tert-butyle, pentyle ou heptyle.
- le terme radical alkyloxy linéaire ou ramifié désigne de préférence les radicaux méthoxy ou éthoxy, mais peut aussi représenter un radical propoxy, isopropoxy, butoxy linéaire, secondaire ou tertiaire,
- le terme radical alkényle linéaire ou ramifié désigne de préférence un radical vinyle, allyle, 1-propényle, butényle ou pentényle,
- le terme radical acyle ayant de 2 à 8 atomes de carbone désigne de préférence un radical acétyle, propionyle, butyryle ou benzoyle, mais également un radical valéryle, hexanoyle, acryloyle, crotonoyle ou carbamoyle,
- le terme radical carboxy estérifié désigne de préférence un groupe alkyloxy ou (arylalkyl) oxy inférieur carbonyle tel que méthoxycarbonyle, éthoxycarbonyle ou benzyloxycarbonyle,
- le terme radical carbamoyle substitué désigne un groupe N-(monoalkyl inférieur) carbamoyle, par exemple, N-méthylcarbamoyle, N-éthylcarbamoyle ; un groupe N,N-(dialkyl inférieur) carbamoyle, par exemple, N,N-diméthylcarbamoyle, N,N-diéthylcarbamoyle ; un groupe N-(hydroxyalkyl inférieur) carbamoyle, par exemple, N-(hydroxyméthyl) carbamoyle, N-(hydroxyéthyl) carbamoyle ;

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glycxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Le ou les éventuels substituants des radicaux alkyle, alkényle, ou alkyloxy sont choisis, de préférence, dans le groupe formé par les radicaux hydroxyle ; alkyle et alkyloxy linéaire ou ramifié, par exemple méthyle, éthyle, tert-butyle, méthoxy, éthoxy, isopropoxy ; alkyle substitué par un radical alkyloxy tel que, par exemple, méthoxy, éthoxy ou isopropoxy ; amino substitué, tel que monoalkyl- et dialkylamino, par exemple méthylamino, éthylamino ou diméthylamino; carboxy libre ou estérifié, par exemple méthoxycarbonyle ou éthoxycarbonyle, un groupe (carbamoylalkyle inférieur), par exemple carbamoylméthyle, carbamoyléthyle.

Parmi les produits objet de l'invention, peuvent être cités tout particulièrement

- le maléate de [16alpha (±)]-11-éthényl-20,21-dinoréburnaménine,
- le maléate de [16alpha (±)]-11-acétyl-20,21-dinoréburnaménine,
- le fumarate de [16alpha (±)]-alpha-méthyl-20,21-dinoréburnaménin-11-éthanol,
- le [16alpha (±)]-20,21-dinoréburnaménin-11-carboxamide.

L'invention a également pour objet un procédé de préparation des produits de formule (I) telle que définie ci-dessus, caractérisé en ce que l'on soumet un produit de formule (II) :

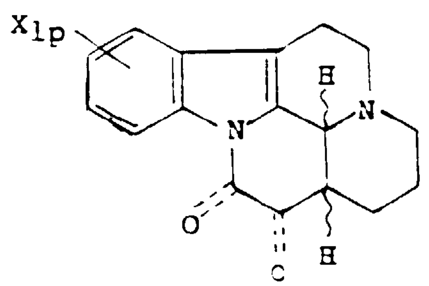

(II)

dans laquelle ==O indique que la seule fonction oxo peut se trouver en position 14 ou 15, et $X_{1p}$, représent $X_1$, qui a les significations indiquées ci-dessus, dans lesquelles les fonctions réactives sont éventuellement protégées, à une réaction de réduction suivie d'une déshydratation et, si nécessaire, d'une élimination des groupements protecteurs des fonctions réactives que peut porter $X_{1p}$, pour obtenir les produits de formule (I) telle que définie ci-dessus, et traite, si désiré les produits de formules (I) par un acide minéral ou organique, lesdits produits de formule (I) étant sous toutes les formes isomères possibles racémiques ou énantiomères.

L'invention a aussi pour objet un procédé de préparation des produits de formule (I) telle que définie ci-dessus, caractérisé en ce que

ou bien l'on soumet un produit de formule (II') :

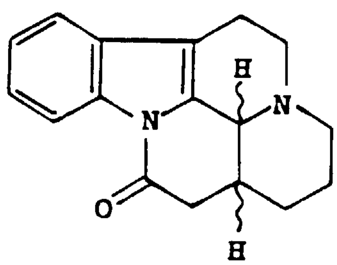

(II')

a) **soit** à une réaction d'halogénation pour obtenir les produits de formule (III) :

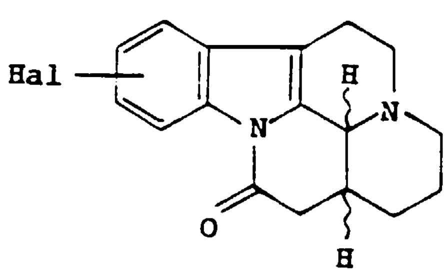

(III)

dans laquelle Hal représente un atome d'halogène, produit de formule (III) que l'on soumet dans un ordre quelconque aux réactions suivantes :

- une réaction de réduction de la fonction oxo suivie de déshydratation,
- une réaction de substitution de l'atome d'halogène pour obtenir les produits de formule ($I'_a$) :

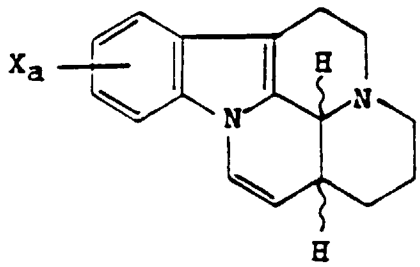

($I'_a$),

dans laquelle $X_a$ a la signification indiquée ci-dessus pour $X_1$ produits de formule ($I'_a$) que, dans le cas où $X_a$ représente un radical formyle, l'on peut soumettre, si désiré, à l'une quelconque des réactions de transformation de ce radical formyle en radical carbamoyle, hydroxyméthyle, ou cyano

b) **soit** à une réaction de nitration,
pour obtenir les produits de formule (IV) :

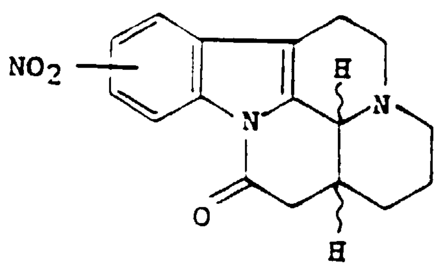

(IV)

que l'on soumet à une réaction de réduction du radical nitro en radical amino,
pour obtenir les produits de formule (V) :

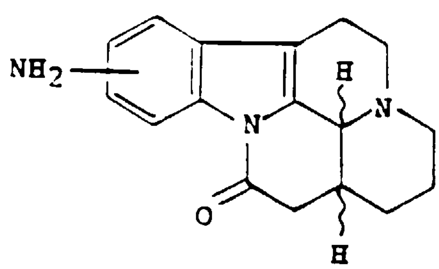

(V)

produit de formule (V) que l'on soumet à la réaction de substitution de l'amine par un atome d'halogéne via les sels de diazonium correspondants pour obtenir les produits de formule (III) telle que définie ci-dessus,

ou bien l'on soumet un produit de formule (II") :

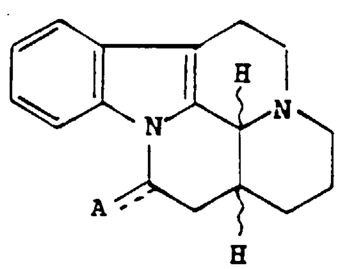

(II")

dans laquelle $\overline{\cdots}$A représente soit une fonction oxo soit une fonction

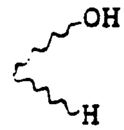 ,

à l'action d'un composé de formule (IX) :

$$X_c - Hal \qquad (IX)$$

dans laquelle Hal représente un atome d'halogène et $X_c$ représente un radical alkényle ou acyle tel que défini ci-dessus pour $X_1$, dans laquelle les fonctions réactives sont éventuellement protégées,
pour obtenir des produits de formule (X) :

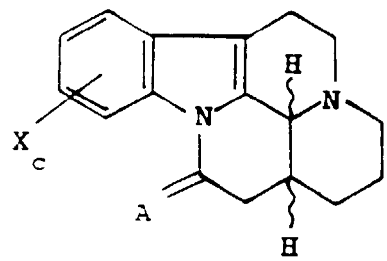

(X)

dans laquelle $X_c$ et A ont la signification indiquée ci-dessus, puis soumet les produits de formule (X) à une réaction de réauction suivie de déshydratation lorsque =A représente une fonction oxo ou seulement à une déshydratation lorsque =A représente une fonction

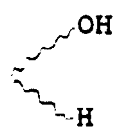

pour obtenir, après élimination, si nécessaire et si désiré, des éventuels groupements protecteurs des fonctions réactives,
les produits de formule ($I'_C$) :

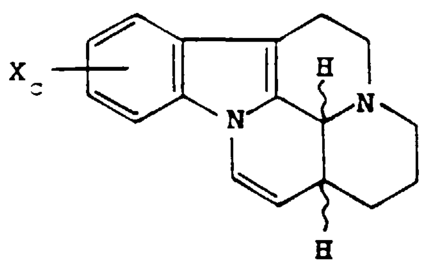

($I'_c$)

dans laquelle $X_c$ a la signification indiquée ci-dessus,
et traite, si désiré les produits de formules ($I'_a$), ($I'_b$), et ($I'_c$) par un acide minéral ou organique,
lesdits produits de formules ($I'_a$), ($I'_b$) et ($I'_c$) étant sous toutes les formes isomères possibles racémiques ou énantiomères.

Dans des conditions préférentielles de mise en oeuvre de l'invention, le procédé ci-dessus est réalisé de la manière suivante :

La réaction de réduction suivie de déshydratation du composé de formule (II) telle que définie ci-dessus peut être réalisée selon les méthodes classiques connues de l'homme de métier : ainsi la réaction de réduction de la fonction cétone en fonction alcool peut êre effectuée, par exemple, dans du borohydrure de sodium ou du cyanoborohydrure de sodium, en présence ou non de chlorure de lithium dans un solvant tel que, par exemple, du méthanol ou de l'éthanol ;

la réaction de déshydratation du composé réduit peut être effectuée, par exemple, soit dans un milieu acide tel que, par exemple, de l'acide chlorhydrique ou de l'acide paratoluène-sulfonique, soit en présence de trifluoroacétate

de cuivre, dans des solvants tels que benzène, toluène, xylène.

La réaction d'halogénation du produit de formule (II') est effectuée selon les réactions classiques telles que par un acide minéral, préférentiellement l'acide bromhydrique, en présence par exemple, d'eau oxygénée, environ à 60°C, dans les produits de formule (III) obtenus, l'atome d'halogène occupe préférentiellement la position 9 ou 11. Si on obtient un mélange de produits de formule (III) dans lesquels l'atome d'halogène occupe respectivement la position 9 ou 11, ces produits peuvent être séparés par les méthodes classiques telles que par exemple la chromatographie.

Les produits halogénés de formule (III) peuvent être soumis dans un ordre quelconque, à la réaction de réduction-déshydratation et à la réaction de substitution de l'atome d'halogène.

Lorsque les produits de formule (III) sont soumis d'abord à une réduction de la fonction oxo puis à une déshydratation ; ces réactions sont réalisées comme indiqué ci-dessus pour le produit de formule (II).

Les produits déshydratés sont ensuite soumis à une substitution de l'atome d'halogène par le radical $X_a$ : cette réaction peut être réalisée, par exemple, à l'aide d'un organomagnésien de formule $X_a$-Mg-Hal ou d'un organolithien de formule $X_a$-Li préparé selon les conditions habituelles connues de l'homme de métier.

L'atome d'halogène de l'halogénure de magnésium peut être un atome de brome comme par exemple dans le bromure de phénylmagnésium ou encore le bromure d'allyl magnésium mais également un atome d'iode ou encore de chlore.

La préparation de l'halogénure de magnésium peut être réalisée, par exemple, par réaction du magnésium avec un halogénure organique dans un milieu inerte légèrement polaire tel que l'éther selon le procédé de préparation des halogénures organomagnésiens ou réactifs de Grignard.

La réaction du produit déshydraté, avec l'halogénure de magnésium s'effectue préférentiellement dans un solvant organique tel que, par exemple, le tétrahydrofuranne ou l'éther pendant un temps variable allant d'environ deux heures dans le cas où $X_a$ représente un radical allyle à environ 10 heures dans le cas où $X_a$ représente un radical phényle. La réaction est avantageusement effectuée en présence d'un catalyseur tel que, par exemple, du palladium ou du nickel.

La préparation de l'organolithien peut être réalisée par exemple, par l'intermédiaire d'une base forte telle que l'amidure de diisopropyllithium ou préférentiellement le n-butyllithium dans de l'éther éthylique ou du tétrahydrofuranne à basse température, de -70°C à -10°C. La réaction de substitution peut s'effectuer par exemple, par action d'un halogénure, d'un carbonate ou d'un amide approprié dérivé de $X_a$.

Lorsque les produits de formule (III) sont d'abord soumis à une réaction de substitution de l'atome d'halogène, il s'agit avantageusement d'une substitution de l'atome d'halogène par un radical alkényle en utilisant, par exemple, un dérivé correspondant de l'étain, tel que :

$$X_a' - Sn(R_6)_3$$

dans laquelle $X_a'$ représente un radical alkényle tel que défini ci-dessus et $R_6$ représente un radical alkyle renfermant au plus 5 atomes de carbone ; il peut s'agir par exemple, du vinyl tributylétain.

La réaction s'effectue préférentiellement dans un solvant organique tel que, par exemple, le toluène en présence d'un catalyseur tel que, par exemple, le palladium sous agitation et au reflux.

Les produits substitués sont ensuite soumis à une réaction de réduction de la fonction oxo puis à une déshydratation selon des méthodes classiques, en opérant par exemple comme indiqué ci-dessus pour le produit de formule (II).

Les produits de formule ($I'_a$) obtenus peuvent être soumis, si désiré, à des réactions classiques connues de l'homme de métier : oxydation, réduction ou encore substitution du radical $X_a$ ; ainsi, en particulier, le radical formyle que peut représenter $X_a$ peut être transformé, par exemple, en radical hydroxyméthyle, cyano, carbamoyle ou encore carboxyle libre ou estérifié selon les méthodes habituelles.

La réaction de nitration du produit de formule (II') pour donner les produits correspondants de formule (IV) peut être réalisée, par exemple, par un mélange (1/1) d'acide nitrique fumant et d'acide acétique glacial.

La réaction de réduction du radical nitro en radical amino pour obtenir des produits de formule (V), peut être effectuée, par exemple, par hydrogénation catalytique en présence de platine dans un solvant organique tel que, par exemple, un mélange (1/1) d'éthanol et d'acétate d'éthyle.

La réaction de substitution du radical amino du produit de formule (V), par un atome d'halogène, de préférence un atome de brome, peut être réalisée par des réactions classiques connues de l'homme de métier telles que, par exemple, la réaction de Sandmeyer, via les sels de diazonium correspondants.

Les produits de formule (III) ainsi obtenus peuvent être soumis aux réactions indiquées ci-dessus pour donner des produits correspondants de formule ($I'_a$).

La réaction du produit de formule (II") avec un dérivé halogéné de formule (IX) peut être réalisée par les méthodes classiques connues de l'homme de métier par exemple, en présence d'un acide de Lewis tel que du chlorure d'aluminium soit dans un solvant tel que l'anhydride dithiocarbonique soit en solution dans le réactif.

Les produits de formule (X) sont ensuite soumis à une réaction de réduction suivie de déshydratation de la fonction oxo ou seulement à une déshydratation pour donner des produits de formule ($I'_c$), ces réactions pouvant être réalisées

dans les conditions indiquées ci-dessus.

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :

- les groupements hydroxy peuvent être protégés par exemple par les radicaux triméthylsilyle, dihydropyranyle ou méthoxyméthyle,
- les groupements amino peuvent être protégés par exemple par les radicaux trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les groupements carboxy peuvent être protégés par exemple sous forme d'esters formés avec les esters facilement clivables tels que les esters benzyliques ou tert-butyliques ou des esters connus dans la chimie des peptides.

L'élimination de ces groupements protecteurs est effectuée dans les conditions usuelles connues de l'homme de métier notamment l'hydrolyse acide effectuée avec un acide tel que l'acide chlorhydrique, benzène sulfonique ou para-toluène sulfonique, formique ou trifluoroacétique.

Le groupement phtalimido est éliminé par l'hydrazine.

On trouvera une liste de différents groupements protecteurs utilisables par exemple dans le brevet BF 2 499 995.

Les formes optiquement actives des produits de formule (I) peuvent être préparées par dédoublement des racémiques selon les méthodes usuelles.

Les composés de formule (I) tels que définis ci-dessus ainsi que leurs sels d'addition avec les acides présentent d'intéressantes propriétés pharmacologiques.

Certains produits présentent notamment une affinité pour les récepteurs alpha 2 adrénergique.

Certains produits peuvent aussi présenter d'intéressantes propriétés anti-amnésiantes activatrices des fonctions cognitives, anti-dépressives protectrices neuronales, anti-anoxiques, anti-ischémiques.

Les propriétés justifient leur application en thérapeutique et l'invention a également pour objet à titre de médicaments, les produits tels que définis par la formule (I) ci-dessus, lesdits produits de formule (I) étant sous toutes les formes isomères possibles racémiques ou optiquement actives, ainsi que les sels d'addition avec les acides minéraux ou organiques pharmaceutiquement acceptables desdits produits de formule (I).

La présente invention a particulièrement pour objet à titre de médicaments, les produits de formule (I) tels que définis ci-dessus, ainsi que leurs sels d'addition avec les acides minéraux ou organiques pharmaceutiquement acceptables.

L'invention a plus particulièrement pour objet, à titre de médicament, les produits de formule (I) suivants :

- le maléate [16alpha (±)]-11-éthényl-20,21-dinoréburnaménine,
- le maléate de [16alpha (±)]-11-acétyl-20,21-dinoréburnaménine,
- le fumarate de [16alpha (±)]-alpha-méthyl-20,21-dinoréburnaménin-11-méthanol,
- le [16alpha (±)]-20,21-dinoréburnaménin-11-carboxamide.

Les médicaments, objet de l'invention, peuvent être utilisés dans le traitement des insuffisances cérébrales d'origine anoxique ou ischémique dans les troubles de la mémoire et de l'attention. Ils peuvent également être utilisés comme anti-dépresseurs.

La présente invention a également pour objet les compositions pharmaceutiques contenant à titre de principe actif, l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 50 mg par jour chez l'adulte, par voie orale.

Les produits de formule ($II_A$) correspondant aux produits de formule (II) telle que définie ci-dessus dans laquelle ==O se trouve en position 14, peuvent être préparées comme indiqué dans le brevet français n° 2 168 853 à partir des dérivés substitués de la 2,3,4,6,7,12-hexahydro indolo (2,3-a) quinolizine.

Les produits de formule ($II_B$) correspondant aux produits de formule (II) telle que définie ci-dessus dans laquelle ==O se trouve en position 15, peuvent être préparés comme indiqué dans la demande de brevet BF. 89-13544 déposé le 17/10/1989.

Le composé de formule ($II_B$) dans laquelle $X_1$ représente un atome d'hydrogène, est décrit dans le brevet européen n° 0 013 315.

Les composés de départ de formule ($II'_B$) :

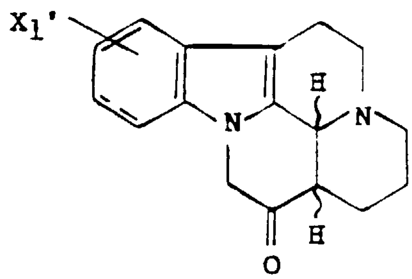

($II'_B$)

dans laquelle $X_1$' représente $X_1$ peuvent être préparés selon le procédé décrit dans le brevet cité précédemment à partir des tryptamines substituées correspondantes.

Un autre procédé de préparation de produits de formule ($II'_B$) telle que définie ci-dessus consiste à soumettre le produit de formule (II'a) :

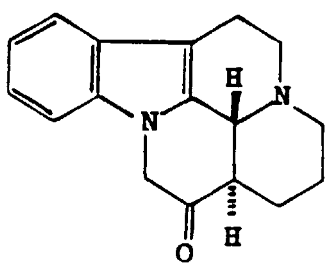

(II'a)

à une réaction de nitration pour obtenir un produit de formule (II'b) :

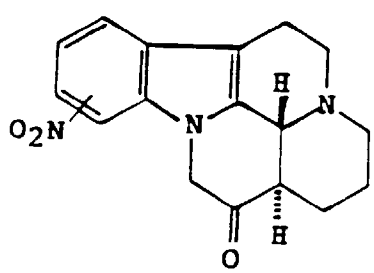

(II'b)

,

que l'on réduit le cas échéant pour obtenir un produit de formule (II'c) :

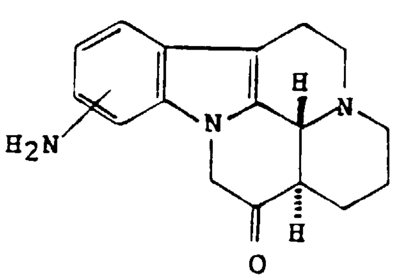

(II'c)

que, le cas échéant l'on transforme en sel de diazonium à partir duquel on prépare par les procédés connus les dérivés de formule (II'd):

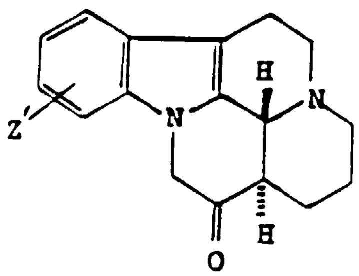

(II'd)
,

dans laquelle Z' représente un atome d'halogène ou un radical hydroxy ou phényle éventuellement substitué, que l'on transforme le cas échéant en dérivés correspondants dans lesquels Z représente un radical alkyloxy

Certains produits de formule ($II_B$) sont nouveaux et la présente invention a encore pour objet à titre de produits industriels nouveaux, les composés de formule ($II"_B$) :

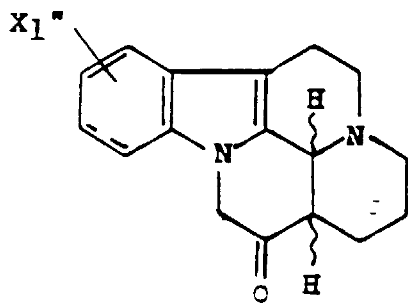

($II"_B$)

dans laquelle $X_1$", est choisi dans le groupe formé par les radicaux cyano, formyle, acyle ou alcoxycarbonyle ayant de 2 à 4 atomes de carbone ou carbamoyle

Les produits de formule (II') et (II") sont des produits connus ; ils peuvent être préparés comme indiqué dans le brevet belge 764 166 et les brevets français 2 190 113 et 2 381 048.

La présente invention a enfin pour objet, à titre de produits industriels nouveaux, et notamment à titre de produits intermédiaires nécessaire à la préparation des produits de formule (I), les composés de formules (VIII) et (X).

Les exemples suivants illustrent l'invention sans toutefois la limiter.

**<u>EXEMPLE 1</u> : Maléate de [16alpha (±)]-11-éthényl-20,21-dinoréburnaménine,**

<u>Stade A</u> : [16alpha (±)]-11-éthényl-20,21-dinoréburnaménin-14(15H)-one.

A une solution de 3 g de [16alpha (±)]-11-bromo-20,21-dinoréburnaménin-14(15H)-one (obtenu comme dans le brevet belge 44087 B) et 300 $cm^3$ de toluène, on ajoute 2,9 $cm^3$ de vinyl tributyl étain puis 100 mg de tétrakis (triphénylphosphine) palladium. On agite 24 heures au reflux. On filtre l'insoluble et amène à sec le filtrat sous pression réduite. On chromatographie le résidu sur silice (éluant : acétate d'éthyle pur). On obtient 2,1 g de produit attendu F = 164°C.

Spectre IR = $CHCl_3$

| | |
|---|---|
| C=O | 1704 $cm^{-1}$ |
| C=C + aromatique | 1651 $cm^{-1}$<br>1628 $cm^{-1}$<br>1612 $cm^{-1}$<br>1565 $cm^{-1}$<br>1478 $cm^{-1}$ |

<u>Stade B</u> : [16alpha (±)]-14,15-dihydro-11-éthényl 20,21-dinoréburnaménin-14-ol

A une solution de 1 g du produit obtenu au stade A dans 50 $cm^3$ de méthanol, on ajoute lentement 325 mg d'hydrure

de bore et de sodium, puis 288 mg de chlorure de lithium. On agite pendant 6 heures en maintenant le reflux, refroidit puis ajoute 100 $cm^3$ d'eau glacée. On agite pendant 30 minutes, essore, lave à l'eau. On obtient 800 mg de produit attendu. F = 195°C.
Spectre IR = $CHCl_3$ peu ou pas de C=O

| | |
|---|---|
| -OH | 3584 $cm^{-1}$ |
| C=C + aromatique | 1627 $cm^{-1}$<br>1602 $cm^{-1}$<br>1552 $cm^{-1}$ |

Stade C : Maléate de [16alpha (±)]-11-éthényl-20,21-dinoréburnaménine

A une solution de 780 mg du produit obtenu au stade B dans 50 $cm^3$ de toluène chaud, on ajoute 16 mg d'acide paratoluène sulfonique. On agite 15 heures au reflux, on refroidit, filtre et amène le filtrat à sec sous pression réduite. On chromatographie le résidu sur silice (éluant : acétate d'éthyle) et on obtient 480 mg de produit recherché. F = 136°C.
Salification : On dissout 457 mg de la base, obtenue ci-dessus, dans 50 $cm^3$ d'acétate d'éthyle à chaud puis on ajoute une solution de 192 mg d'acide maléique dans 30 $cm^3$ d'acétate d'éthyle bouillant. On agite 3 heures en laissant revenir à température ambiante, essore, lave et sèche à 50°C sous pression réduite. On obtient 600 mg du produit recherché. F = 210°C.
Spectre IR = $CHCl_3$ (sur la base)

| | |
|---|---|
| -C=C-<br>C=$CH_2$<br>aromatique | 1649 $cm^{-1}$<br>1627 $cm^{-1}$<br>1613 $cm^{-1}$<br>1554 $cm^{-1}$<br>1477 $cm^{-1}$ |

| Analyse pour : $C_{23}H_{24}N_2O_4$ | | | |
|---|---|---|---|
| calculés : | C% 70,39 | H% 6,16 | N% 7,14 |
| trouvés : | 70,1 | 6,0 | 7,0 |

**<u>EXEMPLE 2</u> : Maléate acide de [16alpha (±)]-11-phényl-20,21-dinoréburnaménine**

A une solution de 580 mg de [16alpha (±)]-11-bromo 20,21-dinoréburnaménine (préparé selon BF 2.623.501) dans 15 $cm^3$ de tétrahydrofuranne, on ajoute 60 mg de chlorure de nickel diphénylphosphinopropane, on chauffe à 50°C puis on ajoute 4,4 $cm^3$ d'une solution 0,8 M de bromure de phényl magnésium dans le tétrahydrofuranne. On maintient 5 heures dans ces conditions. On verse dans 50 $cm^3$ d'eau glacée, extrait avec de l'acétate d'éthyle, lave à l'eau, sèche et amène à sec sous pression réduite. On chromatographie le résidu sur silice (éluant : chlorure de méthylène-acétonitrile (8-2)). On obtient 510 mg du produit attendu (F = 145°C).
**Salification** : A une solution de 560 mg de base (obtenue comme ci-dessus) dans 40 $cm^3$ d'acétate d'éthyle et 10 $cm^3$ d'éthanol, on ajoute 200 mg d'acide maléique en solution dans 10 $cm^3$ d'acétate d'éthyle bouillant. On agite 5 heures à température ambiante. On essore, sèche à 70°C sous pression réduite et recueille 585 mg de produit attendu (F = 230°C).
Spectre IR = $CHCl_3$

| | |
|---|---|
| C=C + aromatique | 1650 $cm^{-1}$<br>1612 $cm^{-1}$<br>1600 $cm^{-1}$<br>1555 $cm^{-1}$<br>1499 $cm^{-1}$ |

| Analyse pour : $C_{27}H_{26}N_2O_4$ = 442,52 | | | |
|---|---|---|---|
| calculés : | C% 73,28 | H% 5,92 | N% 6,33 |
| trouvés : | 73,0 | 5,7 | 6,4 |

**<u>EXEMPLE 3</u> : Maléate acide de [16alpha (±)]-11-(2-propényl) 20,21-dinoréburnaménine**

On opère comme à l'exemple 2 à partir de 1 g de [16alpha (±)]-11-bromo-20,21-dinoréburnaménine (préparé selon BF 2.623.501) en utilisant 4 $cm^3$ de bromure d'allyl magnésium en solution 1M dans le tétrahydrofuranne. On obtient après chromatographie sur silice (éluant : chlorure de méthylène-acétonitrile (8-2)) 450 mg de produit recherché. F ≤ 70°C sous forme de base.
**Salification** : 430 mg de la base sont salifiés en opérant comme à l'exemple 2. On obtient 525 mg du maléate attendu. F = 170°C.
Spectre IR = $CHCl_3$

| | |
|---|---|
| -C=C- + aromatique | 1651 $cm^{-1}$<br>1612 $cm^{-1}$<br>1560 $cm^{-1}$ |
| $=CH_2$ | 1636 $cm^{-1}$<br>919 $cm^{-1}$ |

Bandes de Bohlmann.

| Analyse pour : $C_{24}H_{26}N_2O_4$ = 406,486 | | | |
|---|---|---|---|
| calculés : | C% 70,92 | H% 6,45 | N% 6,89 |
| trouvés : | 70,8 | 6,4 | 6,8 |

**<u>EXEMPLE 4</u> : Maléate acide de [16alpha (±)]-11-acétyl-20,21-dinoréburnaménine**

A une suspension de 4,03 g de [16alpha (±)]-20,21-dinoréburnaménin-14(15H)-one, préparée selon BF 2.381.048 et 100 $cm^3$ de chlorure d'acétyle on ajoute à 0°C, 8 g de chlorure d'aluminium, agite 1 heure à température ambiante puis verse dans l'eau glacée. On alcalinise par ajout d'ammoniaque concentrée, extrait avec de l'acétate d'éthyle, lave à l'eau, sèche et évapore à sec sous pression réduite. On reprend le résidu avec 200 $cm^3$ de toluène et ajoute 200 mg d'acide paratoluène sulfonique. On chauffe au reflux pendant 15 heures. On filtre l'insoluble et évapore le filtrat à sec sous pression réduite. On chromatographie le résidu sur silice (éluant : acétate d'éthyle-éther isopropylique (8-2)) et obtient 1,47 g de produit recherché (F = 144°C) sous forme de base.
**Salification** : A une solution de 750 mg de la base obtenue ci-dessus dans un mélange de 50 $cm^3$ d'acétate déthyle et 50 $cm^3$ d'éthanol on ajoute une solution de 294 mg d'acide maléique dans 20 $cm^3$ d'éthanol. On agite quelques minutes, essore et sèche sous pression réduite à 80°C. On obtient 820 mg de produit attendu. F = 246°C.
Spectre IR = $CHCl_3$

| | |
|---|---|
| >= O | 1667 $cm^{-1}$ |
| -C=C- + aromatique | 1646 $cm^{-1}$<br>1613 $cm^{-1}$<br>1600 $cm^{-1}$<br>1554 $cm^{-1}$ |

| Analyse pour : $C_{23}H_{24}N_2O_5$ = 408,457 | | | |
|---|---|---|---|
| calculés : | C% 67,64 | H% 5,92 | N% 6,86 |
| trouvés : | 67,5 | 5,9 | 6,8 |

**EXEMPLE 5 : Fumarate acide de [16alpha (±)]-alpha-méthyl 20,21-dinoréburnaménin-11-méthanol**

A une suspension de 1,3 g de [16alpha (±)]-1-(20,21-dinoréburnaménin-11-yl) éthanone, obtenue à l'exemple 4 avec 30 $cm^3$ de méthanol, on ajoute lentement 850 mg d'hydrure de bore et de sodium. On agite 2 heures à température ambiante et ajoute 100 $cm^3$ d'eau glacée. On essore, lave à l'eau, sèche à 85°C sous pression réduite. On obtient après purification par empâtage dans l'acétone, 1,01 g du produit recherché (F = 214°C) sous forme de base.
**Salification** : 1 g de la base obtenue est dissous dans un mélange de 50 $cm^3$ d'éthanol et 100 $cm^3$ d'acétate d'éthyle, on ajoute une solution de 394 mg d'acide fumarique dans 20 $cm^3$ d'éthanol bouillant. On agite 2 heures à 0°C, essore et obtient 930 mg de produit attendu. F = 225°C.
Spectre IR =

C=C + aromatique: 1649 $cm^{-1}$, 1612 $cm^{-1}$, 1560 $cm^{-1}$

| Analyse pour : $C_{23}H_{26}N_2O_5$ = 410,473 | | | |
|---|---|---|---|
| calculés : | C% 67,3 | H% 6,38 | N% 6,82 |
| trouvés : | 67,3 | 6,4 | 6,7 |

**EXEMPLE 6 : Maléate acide de [16alpha (±)]-20,21-dinoréburnaménin-11-carboxaldéhyde**

A une solution refroidie à -70°C de 3 g de [16alpha (±)]-11-bromo 20,21-dinoréburnaménine (préparé selon BF 2.623.501) dans 50 $cm^3$ de tétrahydrofuranne, on ajoute, 7,3 $cm^3$ de n-butyl lithium en solution 1,6 M dans l'hexane. On agite 45 minutes à -70°C. On ajoute ensuite à -70°C, 1,4 $cm^3$ de diméthylformamide en solution dans 3 $cm^3$ de tétrahydrofuranne. On laisse revenir à 0°C et agite 40 minutes à 0°C. On verse sur une solution aqueuse de chlorure d'ammonium, extrait avec de l'acétate d'éthyle, sèche et évapore à sec sous pression réduite. On chromatographie le résidu sur silice (éluant : acétate d'éthyle). On obtient 2,11 g de produit attendu sous forme de base (F = 132°C).
**Salification** : On dissout 1 g de la base obtenue ci-dessus dans un mélange de 40 $cm^3$ d'éthanol et 60 $cm^3$ d'acétate d'éthyle. On ajoute une solution de 417 mg d'acide maléique dans 20 $cm^3$ d'éthanol. On agite 2 heures à 20°C, essore, lave à l'éthanol, sèche à 70°C sous pression réduite ; on obtient 1,06 g de produit recherché F = 225°C.
Spectre IR =

= O: 1682 $cm^{-1}$
-C=C- + aromatique: 1644 $cm^{-1}$, 1613 $cm^{-1}$, 1596 $cm^{-1}$

1558 $cm^{-1}$

| Analyse pour : $C_{22}H_{22}N_2O_5$ = 394,43 | | | |
|---|---|---|---|
| calculés : | C% 67 | H% 5,62 | N% 7,1 |
| trouvés : | 67 | 5,6 | 7,1 |

**EXEMPLE 7 : Fumarate acide de [16alpha (±)]-20,21-dinoréburnaménin-11-méthanol**

A une solution de 1,1 g de [16alpha (±)]-20,21-dinoréburnaménin-11-carboxaldéhyde, sous forme de base obtenue à l'exemple 6, dans 50 $cm^3$ de méthanol on ajoute, lentement, à 20°C, 341 mg d'hydrure de bore et de sodium et agite pendant 2 heures. On ajoute 200 $cm^3$ d'eau, essore, lave à l'eau puis à l'acétone et obtient 967 mg de produit attendu sous forme de base (F = 214°C).

**Salification** : A une solution de 967 mg de la base ci-dessus dans 120 $cm^3$ d'un mélange acétate d'éthyle-éthanol (1-1), on ajoute ensuite 400 mg d'acétate fumarique en solution dans 20 $cm^3$ d'éthanol bouillant. On essore et obtient 1,1 g du produit attendu (F = 255°C).
Spectre IR = peu ou pas de >= O

| | |
|---|---|
| OH | 3610 $cm^{-1}$ |
| -C=C- + | 1649 $cm^{-1}$ |
| aromatique | 1616 $cm^{-1}$ |

| Analyse pour : $C_{22}H_{24}N_2O_5$ = 396,446 | | | |
|---|---|---|---|
| calculés : | C% 66,65 | H% 6,1 | N% 7,07 |
| trouvés : | 67,0 | 6,3 | 6,9 |

**EXEMPLE 8 : Maléate acide de [16alpha (±)-20,21-dinoréburnaménin-11-carboxylate d'éthyle**

On opère comme à l'exemple 6 à partir de 3,29 g de [16alpha (±)]-11-bromo-20,21-dinoréburnaménine (obtenu selon BF 2.623.501) et en utilisant 1,8 $cm^3$ de carbonate d'éthyle. Après chromatographie sur silice, (éluant acétate d'éthyle - éther isopropylique (8-2)), on obtient 1,36 g de produit attendu sous forme de base (F = 110°C).
**Salification** : A une solution de 600 mg de la base ci-dessus dans 50 $cm^3$ d'acétate d'éthyle et 10 $cm^3$ d'éthanol, on ajoute 216 mg d'acide maléique en solution dans 10 $cm^3$ d'éthanol. On agite quelques instants, essore, lave avec de l'acétate d'éthyle. On obtient 460 mg de produit recherché F = 230°C.
Spectre IR =

| | |
|---|---|
| >= O | 1702 $cm^{-1}$ |
| -C=C- | 1646 $cm^{-1}$ |
| + | 1612 $cm^{-1}$ |
| aromatique | 1560 $cm^{-1}$ |
| | 1477 $cm^{-1}$ |

| Analyse pour : $C_{24}H_{26}N_2O_6$ = 438,48 | | | |
|---|---|---|---|
| calculés : | C% 65,74 | H% 5,98 | N% 6,39 |
| trouvés : | 65,9 | 5,9 | 6,5 |

**EXEMPLE 9 : Maléate acide de [16alpha (±)]-20,21-dinoréburnaménin-11-carbonitrile**

A une solution de 1,4 g de [16alpha (±)]-20,21-dinoréburnaménin-11-carboxaldéhyde (préparée comme à l'exemple 6) dans 50 $cm^3$ d'isopropanol on ajoute, par barbotage jusqu'à saturation, de l'ammoniac gazeux ; en maintenant ce barbotage, on ajoute 8,7 g de bioxyde de manganèse. On agite 3 heures à température ambiante puis ajoute 20 $cm^3$ de chlorure de méthylène et filtre. Le filtrat est amené à sec sous pression réduite. On chromatographie le résidu sur silice (éluant : acétate d'éthyle) et obtient 1,3 g de produit recherché sous forme de base (F = 146°C).
**Salification** : A une solution de 700 mg de la base ci-dessus, dans 40 $cm^3$ d'acétate d'éthyle et 10 $cm^3$ d'éthanol, on ajoute 295 mg d'acide maléique en solution dans 10 $cm^3$ d'éthanol. On agite 2 heures à température ambiante, essore et obtient 830 mg du produit attendu (F = 228°C).
Spectre IR = ($CHCl_3$)

| | |
|---|---|
| -C ≡ N | 2222 $cm^{-1}$ (F) |

Bandes de Bohlmann

| | |
|---|---|
| C=C C=N + aromatique | 1646 $cm^{-1}$<br>1614 $cm^{-1}$<br>1605 $cm^{-1}$<br>1555 $cm^{-1}$ |

| Analyse pour : $C_{22}H_{21}N_3O_4$ = 321,43 | | | |
|---|---|---|---|
| calculés : | C% 67,51 | H% 5,41 | N% 10,73 |
| trouvés : | 67,7 | 5,2 | 10,8 |

**EXEMPLE 10 : [16alpha (±)]-20,21-dinoréburnaménin-11-carboxamide**

A une solution de 600 mg du produit obtenu à l'exemple 9 dans 30 $cm^3$ de méthanol, on ajoute 20 $cm^3$ de soude 5N puis 2 $cm^3$ d'eau oxygénée. On agite pendant 3 heures à température ambiante, ajoute 100 $cm^3$ d'eau, essore, lave à l'eau et sèche 5 à 75°C sous pression réduite. On obtient 580 mg de produit brut (F = 258°C) que l'on empâte dans l'éthanol pour recueillir 410 mg du produit recherché (F = 258°C).

Spectre IR =

| | |
|---|---|
| >= O | 1638 $cm^{-1}$ |
| Aromatique + $NH_2$ + C=C | 1610 $cm^{-1}$<br>1554 $cm^{-1}$ |

| Analyse pour : $C_{18}H_{19}N_3O$ = 293,37 | | | |
|---|---|---|---|
| calculés : | C% 73,7 | H% 6,53 | N% 14,32 |
| trouvés : | 73,4 | 6,3 | 14,2 |

**EXEMPLE 11 : Composition pharmaceutique**

On a préparé des comprimés répondant à la formule suivante :

| | |
|---|---|
| Produit de l'exemple 1 | 25 mg |
| Excipient pour un comprimé terminé à | 300 mg |

(détail de l'excipient : lactose, talc, amidon, stéarate de magnésium)

**ETUDE PHARMACOLOGIQUE** :

1 - Anoxie hypobare.

L'épreuve consiste à mesurer le temps de survie de souris soumises à une atmosphère hypoxique léthale. Pour cela, du souris mâles de type CD1, Charles River de 20 à 25 grammes 5 sont placées individuellement dans une enceinte de 2 litres dans laquelle on réalise au moyen d'une pompe à vide une dépression de 620 mm Hg selon la cinétique suivante :

| Temps (s) dépression (mmHg) | |
|---|---|
| 0 | 0 |

(suite)

| Temps (s) | dépression (mmHg) |
|---|---|
| 3,5 | 350 |
| 6 | 400 |
| 9 | 450 |
| 12 | 500 |
| 16 | 550 |
| 28 | 600 |
| 34 | 610 |
| 55 | 620 |

Le temps de survie est le temps compris entre le début de la dépression (TO) et le dernier mouvement respiratoire ; il est de l'ordre de 70 à 80 s chez les animaux témoins. Les résultats sont donnés en pourcentage d'augmentation du temps de survie par rapport aux animaux témoins qui ont reçu le véhicule.

| Produit de l'exemple | % augmentation du temps de survie |
|---|---|
| 4 | + 22 |
| 5 | + 21 |
| 10 | + 22 |

2 - Affinité pour les récepteurs alpha2 adrénergiques :

On homogénéise dans 90 ml de sucrose 0,32 M, 10 cortex prélevés sur des cerveaux de rats mâles pesant 150 g en moyenne. Après centrifugation à 1 000 g du mélange homogénéisé pendant 10 minutes à 0°C, le surnageant est centrifugé à 30 000 g pendant 10 minutes à 0° + 4°C. Le culot est mis en suspension dans 240 ml de tampon TrisHCl 50 mM pH 7,7 et centrifugé à 30 000 g pendant 15 minutes à 0° + 4°C. Le nouveau culot obtenu est mis en suspension dans 480 ml de tampon $NaKPO_4$ pH 7,4, 50 mM.

On fait ensuite incuber pendant 45 minutes à 25°C, 2 ml de suspension en présence de $^3H$ rauwolscine à la concentration 0,15 nM :

i) seule,
ii) avec des concentrations croissantes du produit à tester ou,
iii) pour déterminer la fixation non spécifique, avec de la phentolamine non radioactive à la concentration $10^{-5}$M.

Les suspensions incubées sont filtrées sur Whatman GF/C et les filtres sont lavés par trois fois 5 ml de tampon $NaKPO_4$ pH 7,4 à 0°C. La radioactivité des filtres est mesurée par scintillement liquide.

L'affinité du produit testé pour les récepteurs alpha2 adrénergiques est donnée relativement à la phentolamine comme produit de référence.

CD = concentration de phentolamine inhibant 50 % de la fixation spécifique de la $^3H$ rauwolscine ;
CX = concentration du produit à tester inhibant 50 % de la fixation spécifique de la $^3H$ rauwolscine.

L'affinité relative est donnée par la relation :

$$ARL = 100\ \frac{CD}{CX}$$

| Résultats produits de l'exemple | ARL |
|---|---|
| 1 | 31 |

3 - Etude de la toxicité aiguë

On a évalué les doses létales $DL_O$ des différents composés testés après administration par voie orale chez la souris.

On appelle $DL_O$ la dose maximale ne provoquant aucune mortalité en 8 jours.

Les résultats obtenus sont les suivants :

| Produit de l'exemple | % augmentation du temps de survie |
|---|---|
| 1 | ≥ 400 |
| 4 | 100 |
| 5 | 100 |

**Revendications**

**Revendications pour les Etats contractants suivants : AT, BE, CH, DE, DK, FR, GB, IT, LI, LU, NL, SE**

1. Produits de formule (I) :

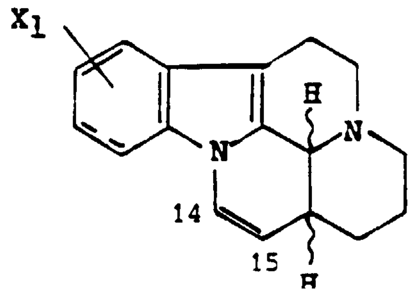

(I)

dans laquelle $X_1$ est choisi parmi les radicaux suivants :

- un radical, alkényle linéaire ou ramifié renfermant au plus 18 atomes de carbone ou hydroxyalkyle ayant au plus 8 atomes de carbone,
- un radical cyano, formyle ou acyle ayant de 2 à 4 atomes de carbone,
- un radical alcoxycarbonyle ayant de 2 à 4 atomes de carbone ou carbamoyle,
- un radical phényle,

lesdits produits de formule (I) étant sous toutes les formes isomères possibles racémiques, énantiomères et diastéréoisomères, ainsi que les sels d'addition avec les acides minéraux et organiques desdits produits de formule (I).

2.
- le maléate de [16alpha (±)]-11-éthényl-20,21-dinoréburnaménine,
- le maléate de [16alpha (±)]-11-acétyl-20,21-dinoréburnaménine,
- le fumarate de [16alpha (±)]-alpha-methyl-20,21-dinoréburnaménin-11-méthanol,
- le [16alpha (±)]-20,21-dinoréburnaménin-11-carboxamide.

3. Procédé de préparation des produits de formule (I) telle que définie à la revendication 1, caractérisé en ce que l'on soumet un produit de formule (II) :

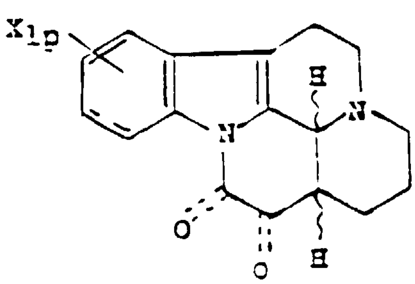

(II)

dans laquelle ==O indique que la seule fonction oxo peut se trouver en position 14 ou 15, et $X_{1p}$, représent $X_1$, qui a la signification indiquée à la revendication 1, dans lesquelles les fonctions réactives sont éventuellement protégées, à une réaction de réduction suivie d'une déshydratation et, si nécessaire, d'une élimination des groupements protecteurs des fonctions réactives que peut porter $X_{1p}$, pour obtenir les produits de formule (I) telle que définie à la revendication 1, et traite, si désiré les produits de formule (I) par un acide minéral ou organique, lesdits produits de formule (I) étant sous toutes les formes isomères possibles racémiques ou énantiomères.

**4.** Procédé de préparation des produits de formule (I) telle que définie à la revendication 1, caractérisé en ce que ou bien l'on soumet un produit de formule (II') :

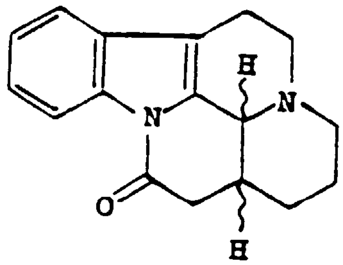

(II')

a) **soit** à une réaction d'halogénation pour obtenir les produits de formule (III) :

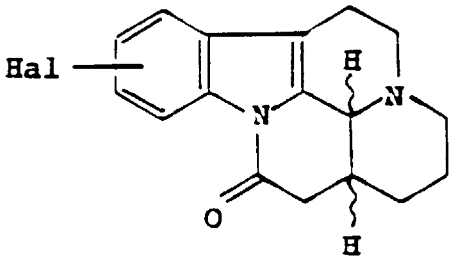

(III)

dans laquelle Hal représente un atome d'halogène, produit de formule (III) que l'on soumet dans un ordre quelconque aux réactions suivantes :

- une réaction de réduction de la fonction oxo suivie de déshydratation,
- une réaction de substitution de l'atome d'halogène pour obtenir les produits de formule ($I'_a$) :

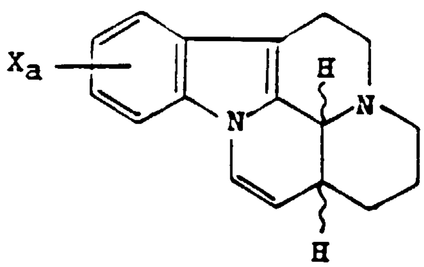

($I'_a$)

dans laquelle $X_a$ a la signification indiquée ci-dessus pour $X_1$, produits de formule ($I'_a$) que, dans le cas où $X_a$ représente un radical formyle, l'on peut soumettre, si désiré, à l'une quelconque des réactions de transformation de ce radical formyle en radical carbamoyle, hydroxyméthyle, ou cyano

b) **soit** à une réaction de nitration, pour obtenir les produits de formule (IV) :

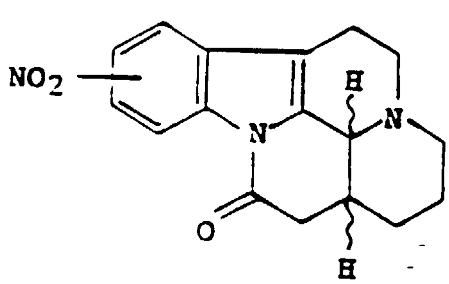

(IV)

que l'on soumet à une réaction de réduction du radical nitro en radical amino, pour obtenir les produits de formule (V) :

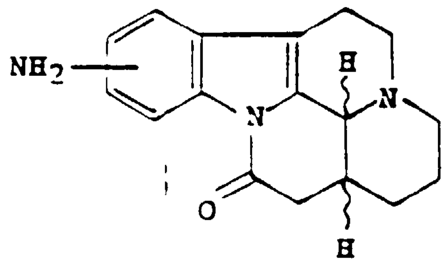

(V)

produit de formule (V) que l'on soumet à la réaction de substitution de l'amine par un atome d'halogène via les sels de diazonium correspondants pour obtenir les produits de formule (III) telle que définie ci-dessus,

ou bien l'on soumet un produit de formule (II") :

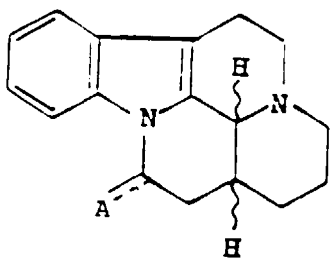

(II")

dans laquelle = A représente soit une fonction oxo soit une fonction

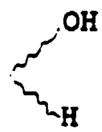

,

à l'action d'un composé de formule (IX) :

$$X_c - \mathrm{Hal} \qquad \text{(IX)}$$

dans laquelle Hal représente un atome d'halogène et $X_c$ représente un radical alkényle ou acyle tel que défini ci-dessus pour $X_1$, dans laquelle les fonctions réactives sont éventuellement protégées,
pour obtenir des produits de formule (X) :

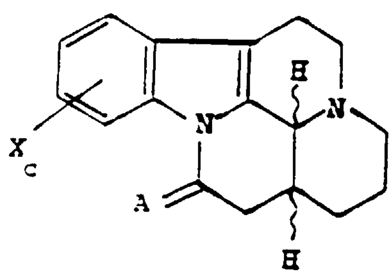

(X)

dans laquelle $X_c$ et A ont la signification indiquée ci-dessus, puis soumet les produits de formule (X) à une réaction de réduction suivie de déshydratation lorsque =A représente une fonction oxo ou seulement à une déshydratation lorsque A représente une fonction

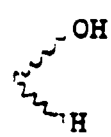

pour obtenir, après élimination, si nécessaire et si désiré, des éventuels groupements protecteurs des fonctions réactives,

les produits de formule ($I'_c$) :

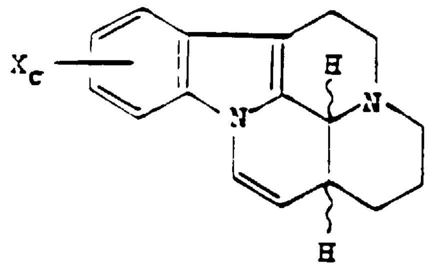

($I'_c$)

dans laquelle $X_c$ a la signification indiquée ci-dessus, et traite, si désiré les produits de formules ($I'_a$), ($I'_b$), et ($I'_c$) par un acide minéral ou organique, lesdits produits de formules ($I'_a$), ($I'_b$) et ($I'_c$) étant sous toutes les formes isomères possibles racémiques ou énantiomères.

**5.** A titre de médicaments, les produits tels que définis par La formule (I) de la revendication 1, sous toutes les formes isomères possibles racémiques ou énantiomères, ainsi que les sels d'addition avec les acides minéraux ou organiques pharmaceutiquement acceptables.

**6.** Les compositions pharmaceutiques contenant à titre de principe actif, l'un au moins des médicaments tels que définis à la revendication 5.

**7.** A titre de produits industriels nouveaux, les composés de formule ($II''_B$) :

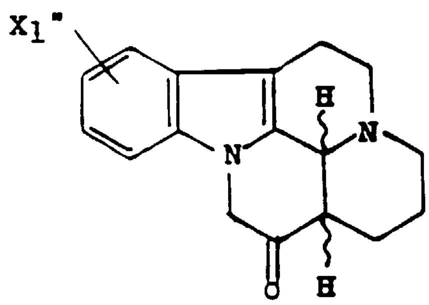

($II''_B$)

dans laquelle $X_1''$, est choisi dans le groupe formé par les radicaux cyano, formyle, acyle ou alcoxycarbonyle ayant de 2 à 4 atomes de carbone ou carbamoyle.

**8.** A titre de produits industriels nouveaux, les composés de formule (X).

**Revendications pour l'Etat contractant suivant : ES**

**1.** Procédé de préparation des produits de formule (I) :

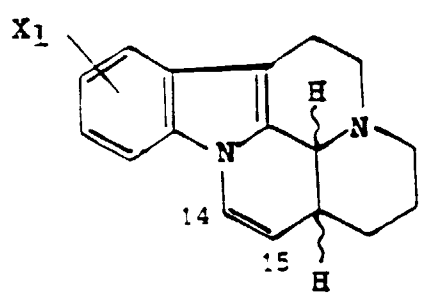

(I)

dans laquelle $X_1$ est choisi parmi les radicaux suivants :

- un radical, alkényle linéaire ou ramifié renfermant au plus 18 atomes de carbone ou hydroxyalkyle ayant au plus 8 atomes de carbone,

- un radical cyano, formyle ou acyle ayant de 2 à 4 atomes de carbone,
- un radical alcoxycarbonyle ayant de 2 à 4 atomes de carbone ou carbamoyle,
- un radical phényle,

lesdits produits de formule (I) étant sous toutes les formes isomères possibles racémiques ou optiquement actives, ainsi que les sels d'addition avec les acides minéraux ou organiques desdits produits de formule (I), caractérisé en ce que l'on soumet un produit de formule (II) :

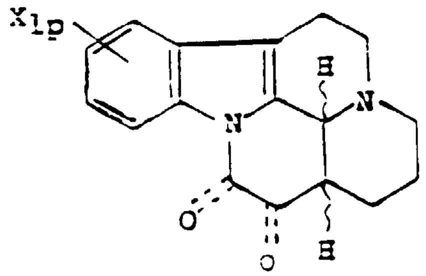

(II)

dans laquelle ==O indique que la seule fonction oxo peut se trouver en position 14 ou 15, et $X_{1p}$ représente $X_1$ qui a la signification indiquée ci-dessus, dans lesquelles les fonctions réactives sont éventuellement protégées, à une réaction de réduction suivie d'une déshydratation et, si nécessaire, d'une élimination des groupements protecteurs des fonctions réactives que peut porter $X_{1p}$ pour obtenir les produits de formule (I) telle que définie ci-dessus, et traite, si désiré les produits de formules (I) par un acide minéral ou organique.

**2.** Procédé selon la revendication 1, caractérisé en ce que l'on utilise au départ un produit de formule (II) dans laquelle la fonction oxo est en position 14.

**3.** Procédé selon la revendication 1, caractérisé en ce que l'on utilise au départ un produit de formule (II) dans laquelle la fonction oxo est en position 15.

**4.** Procédé selon la revendication 1 pour la préparation des produits de formule (I) telle que définie à la revendication 1, caractérisé en ce que ou bien l'on soumet un produit de formule (II') :

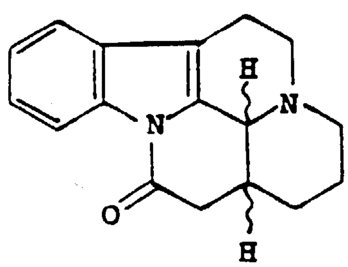

(II')

a) **soit** à une réaction d'halogénation
pour obtenir les produits de formule (III) :

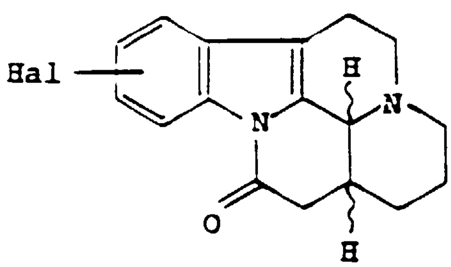

(III)

dans laquelle Hal représente un atome d'halogène, produit de formule (III) que l'on soumet dans un ordre quelconque aux réactions suivantes :

- une réaction de réduction de la fonction oxo suivie de déshydratation,
- une réaction de substitution de l'atome d'halogène pour obtenir les produits de formule ($I'_a$) :

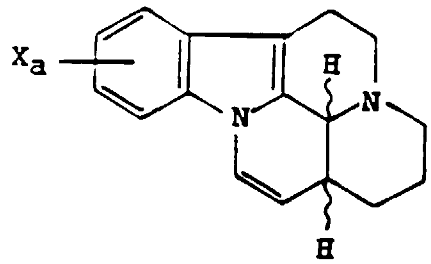

($I'_a$)

dans laquelle $X_a$ a la signification indiqués ci-dessus pour $X_1$, produits de formule ($I'_a$) que, dans le cas où $X_a$ représente un radical formyle, l'on peut soumettre, si désiré, à l'une quelconque des réactions de transformation de ce radical formyle en radical carbamoyle, hydroxyméthyle, ou cyano,

b) **soit** à une réaction de nitration,
pour obtenir les produits de formule (IV) :

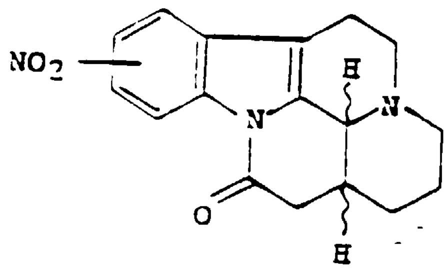

(IV)

que l'on soumet à une réaction de réduction du radical nitro en radical amino,
pour obtenir les produits de formule (V) :

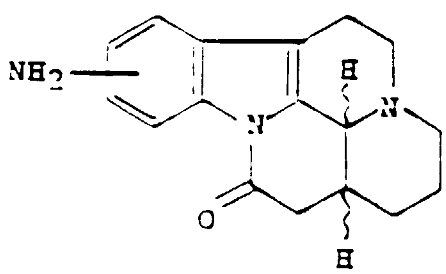

(V)

produit de formule (V) que l'on soumet à la réaction de substitution de l'amine par un atome d'halogène via les sels de diazonium correspondants pour obtenir les produits de formule (III) telle que définie ci-dessus,

<u>ou bien</u> l'on soumet un produit de formule (II") :

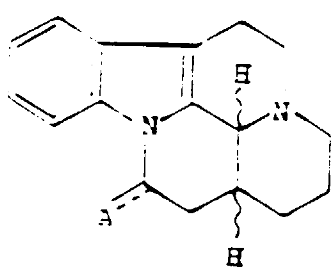

(II")

dans laquelle = A représente soit une fonction oxo soit une fonction

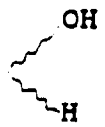 ,

à l'action d'un composé de formule (IX) :

$$X_c - Hal \qquad (IX)$$

dans laquelle Hal représente un atome d'halogène et $X_c$ représente un radical, alkényle ou acyle tel que défini ci-dessus pour $X_1$ dans laquelle les fonctions réactives sont éventuellement protégées,
pour obtenir des produits de formule (X) :

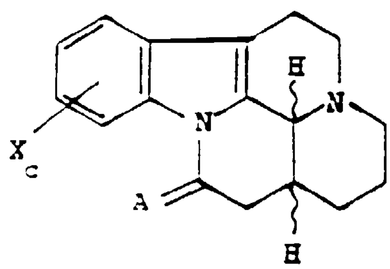

(X)

dans laquelle $X_c$ et A ont la signification indiquée ci-dessus, puis soumet les produits de formule (X) à une réaction de réduction suivie de déshydratation lorsque =A représente une fonction oxo ou seulement à une déshydratation lorsque A représente une fonction

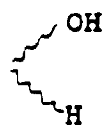

pour obtenir, après élimination, si nécessaire et si désiré, des éventuels groupements protecteurs des fonctions réactives,
les produits de formule ($I'_c$) :

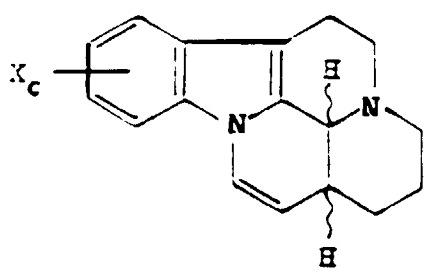

($I'_c$)

dans laquelle $X_c$ a la signification indiquée ci-dessus,
et traite, si désiré les produits de formules ($I'_a$), ($I'_b$), et ($I'_c$) par un acide minéral ou organique,
lesdits produits de formules ($I'_a$) , ($I'_b$) et ($I'_c$) étant sous toutes les formes isomères possibles racémiques ou énantiomères.

**5.** Procédé selon la revendication 1, caractérisé en ce que l'on utilise au départ un produit de formule (II) dans laquelle $X_{1p}$ représente $X_1$, dans lesquels les fonctions réactives sont éventuellement protégées, $X_1$ étant choisi parmi les radicaux suivants :

- un radical, alkényle linéaire ou ramifié renfermant au plus 18 atomes de carbone ou hydroxyalkyle ayant au plus 8 atomes de carbone,
- un radical cyano, formyle ou acyle ayant de 2 à 4 atomes de carbone,
- un radical alcoxycarbonyle ayant de 2 à 4 atomes de carbone ou carbamoyle,
- un radical phényle,

**Revendications pour l'Etat contractant suivant : ES**

1. Procédé de préparation des produits de formule (I) :

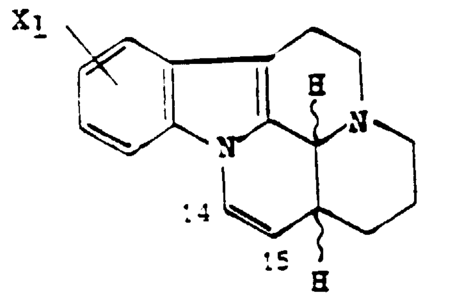

(I)

dans laquelle $X_1$ est choisi parmi les radicaux suivants :

- un radical, alkényle linéaire ou ramifié renfermant au plus 18 atomes de carbone ou hydroxyalkyle ayant au plus 8 atomes de carbone,
- un radical cyano, formyle ou acyle ayant de 2 à 4 atomes de carbone,
- un radical alcoxycarbonyle ayant de 2 à 4 atomes de carbone ou carbamoyle,
- un radical phényle,

lesdits produits de formule (I) étant sous toutes les formes isomères possibles racémiques ou optiquement actives, ainsi que les sels d'addition avec les acides minéraux ou organiques desdits produits de formule (I), caractérisé en ce que l'on soumet un produit de formule (II) :

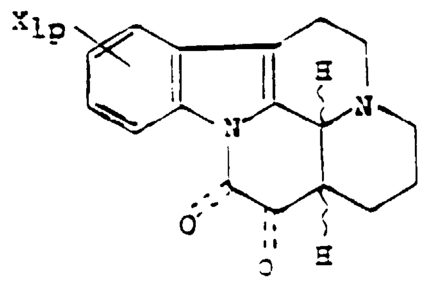

(II)

dans laquelle ==O indique que la seule fonction oxo peut se trouver en position 14 ou 15, et $X_{1p}$ représente $X_1$ qui a la signification indiquée ci-dessus, dans lesquelles les fonctions réactives sont éventuellement protégées, à une réaction de réduction suivie d'une déshydratation et, si nécessaire, d'une élimination des groupements protecteurs des fonctions réactives que peut porter $X_{1p}$ pour obtenir les produits de formule (I) telle que définie ci-dessus, et traite, si désiré les produits de formules (I) par un acide minéral ou organique.

2. Procédé selon la revendication 1, caractérisé en ce que l'on utilise au départ un produit de formule (II) dans laquelle la fonction oxo est en position 14.

3. Procédé selon la revendication 1, caractérisé en ce que l'on utilise au départ un produit de formule (II) dans laquelle la fonction oxo est en position 15.

4. Procédé selon la revendication 1 pour la préparation des produits de formule (I) telle que définie à la revendication 1, caractérisé en ce que <u>ou bien</u> l'on soumet un produit de formule (II') :

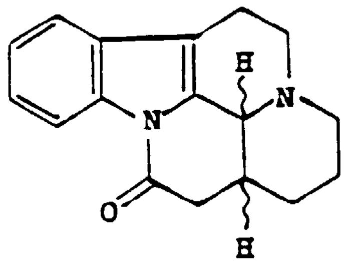

(II')

a) **soit** à une réaction d'halogénation
pour obtenir les produits de formule (III) :

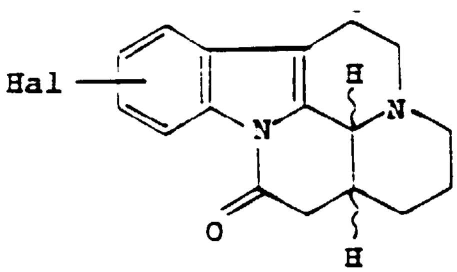

(III)

dans laquelle Hal représente un atome d'halogène, produit de formule (III) que l'on soumet dans un ordre quelconque aux réactions suivantes

- une réaction de réduction de la fonction oxo suivie de déshydratation,
- une réaction de substitution de l'atome d'halogène pour obtenir les produits de formule ($I'_a$) :

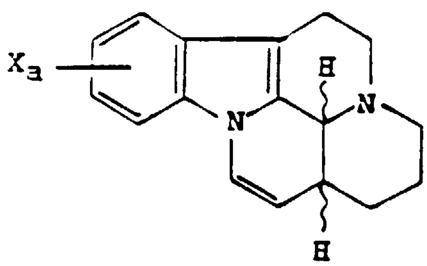

($I'_a$)

dans laquelle $X_a$ a la signification indiquée ci-dessus pour $X_1$, produits de formule ($I'_a$) que, dans le cas où $X_a$ représente un radical formyle, l'on peut soumettre, si désiré, à l'une quelconque des réactions de transformation de ce radical formyle en radical carbamoyle, hydroxyméthyle, ou cyano,

b) **soit** à une réaction de nitration,
pour obtenir les produits de formule (IV) :

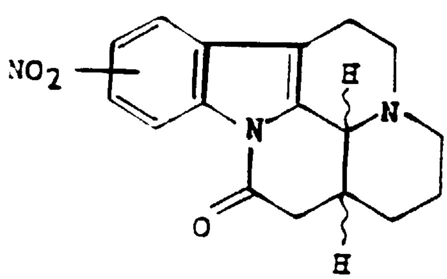

(IV)

que l'on soumet à une réaction de réduction du radical nitro en radical amino,
pour obtenir les produits de formule (V) :

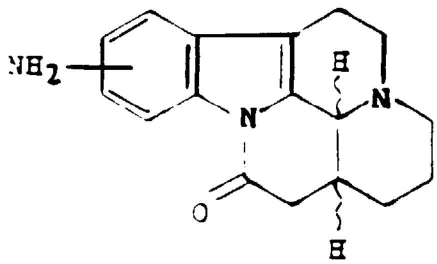

(V)

produit de formule (V) que l'on soumet à la réaction de substitution de l'amine par un atome d'halogène via les sels de diazonium correspondants pour obtenir les produits de formule (III) telle que définie ci-dessus,

<u>ou bien</u> l'on soumet un produit de formule (II") :

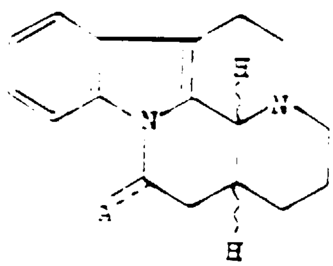

(II")

dans laquelle = A représente soit une fonction oxo soit une fonction

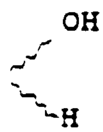

,

à l'action d'un composé de formule (IX) :

$X_c$ - Hal (IX)

dans laquelle Hal représente un atome d'halogène et $X_c$ représente un radical alkényle ou acyle tel que défini ci-dessus pour $X_1$ dans laquelle les fonctions réactives sont éventuellement protégées,
pour obtenir des produits de formule (X) :

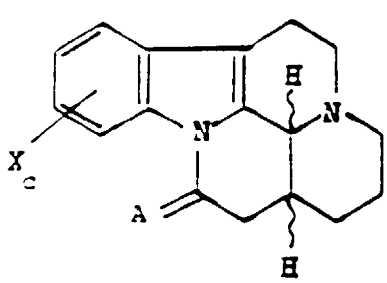

(X)

dans laquelle $X_c$ et A ont la signification indiquée ci-dessus, puis soumet les produits de formule (X) à une réaction de réduction suivie de déshydratation lorsque =A représente une fonction oxo ou seulement à une déshydratation lorsque A représente une fonction

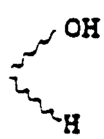

pour obtenir, après élimination, si nécessaire et si désiré, des éventuels groupements protecteurs des fonctions réactives,

les produits de formule (I'$_c$) :

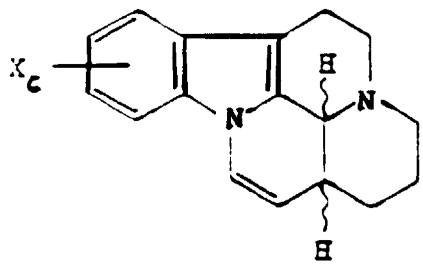

(I'$_c$)

dans laquelle $X_c$ a la signification indiquée ci-dessus, et traite, si désiré les produits de formules (I'$_a$), (I'$_b$), et (I'$_c$) par un acide minéral ou organique, lesdits produits de formules (I'$_a$), (I'$_b$) et (I'$_c$) étant sous toutes les formes isomeres possibles racémiques ou énantiomères.

5. Procédé selon la revendication 1, caractérisé en ce que l'on utilise au départ un produit de formule (II) dans laquelle $X_{1p}$ représente $X_1$, dans lesquels les fonctions réactives sont éventuellement protégées, $X_1$ étant choisi parmi les radicaux suivants :

- un radical, alkényle linéaire ou ramifié renfermant au plus 18 atomes de carbone ou hydroxyalkyle ayant au plus 8 atomes de carbone,
- un radical cyano, formyle ou acyle ayant de 2 à 4 atomes de carbone,
- un radical alcoxycarbonyle ayant de 2 à 4 atomes de carbone ou carbamoyle,
- un radical phényle,

6. Procédé selon l'une quelconque des revendication 1 à 5, caractérisé en ce que l'on prépare :

- le maléate de [16alpha (±)]-11-éthény1-20,21-dinoréburnaménine,
- le maléate de [16alpha (±)]-11-acétyl-20,21-dinoréburnaménine,
- le fumarate de [16alpha (±)]-alpha-méthyl-20,21-dinorébumaménin-11-méthanol,
- le [16alpha (±)]-20,21-dinorébumaménin-11-carboxamide.

7. Procédé de préparation de compositions pharmaceutiques, caractérisé en ce que l'on met à titre de principe actif l'un au moins des produits de formule (I), telle que définie à la revendication 1, sous toutes les formes isomères possibles racémiques et énantiomères ou l'un au moins de leurs sels d'addition avec les acides minéraux ou organiques pharmaceutiquement acceptables, sous une forme destinée à cet usage.

8. Procédé de préparation de compositions pharmaceutiques, caractérisé en ce que l'on met à titre de principe actif l'un au moins des produits de formule (I), telle que définie à la revendication 6, sous toutes les formes isomères possibles racémiques et énantiomères ou l'un au moins de leurs sels d'addition avec les acides minéraux ou organiques pharmaceutiquement acceptables, sous une forme destinée à cet usage.

9. A titre de produits industriels nouveaux, les composés de formule (II"B) :

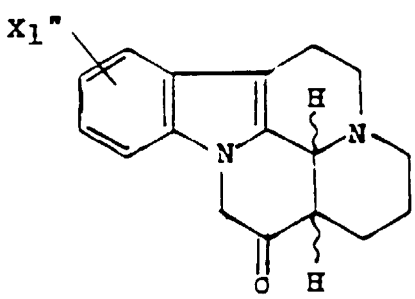

(II"B)

dans laquelle $X_1$" $X_1$" est choisi dans le groupe formé par les radicaux cyano, formyle, acyle ou alcoxycarbonyle ayant de 2 à 4 atomes de carbone ou carbamoyle.

**10.** A titre de produits industriels nouveaux, les composés de formule (X).

## Claims

### Claims for the following Contracting States : AT, BE, CH, DE, DK, FR, GB, IT, LI, LU, NL, SE

**1.** Products of formula (I):

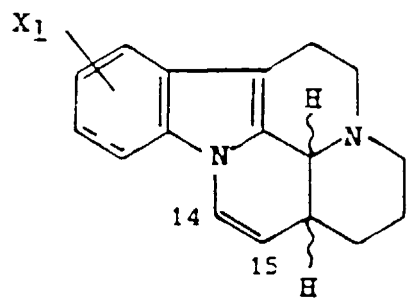

(I)

in which
$X_1$ is chosen from the following radicals:

- a linear or branched alkenyl radical containing at most 18 carbon atoms or a hydroxyalkyl radical having at most 8 carbon atoms,
- a cyano, formyl or acyl radical having 2 to 4 carbon atoms,
- an alkoxycarbonyl radical having 2 to 4 carbon atoms or a carbamoyl radical,
- a phenyl radical,

said products of formula (I) being in all the possible racemic, enantiomeric and diastereoisomeric isomer forms, as well as the addition salts with mineral and organic acids of said products of formula (I).

**2.**
- [16alpha (±)]-11-ethenyl-20,21-dinoreburnamenine maleate,
- [16alpha (±)]-11-acetyl-20,21-dinoreburnamenine maleate,
- [16alpha (±)]-alpha-methyl-20,21-dinoreburnamenin-11-methanol fumarate,
- [16alpha (±)]-20,21-dinoreburnamenin-11-carboxamide.

**3.** Preparation process for the products of formula (I) as defined in claim 1, characterized in that a product of formula (II):

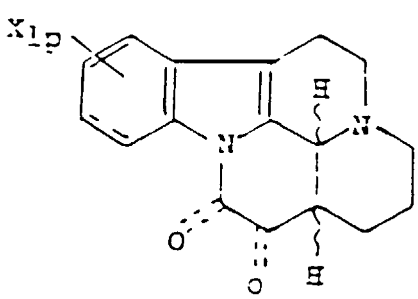

(II)

in which ==O indicates that the single oxo function can be found in position 14 or 15, and $X_{1p}$ represents $X_1$, which has the meaning indicated in claim 1, in which the reactive functions are optionally protected, is subjected to a reduction reaction followed by a dehydration and, if necessary, an elimination of the protective groups of the reactive functions which can be carried by $X_1$p, in order to obtain the products of formula (I) as defined in claim 1, and if desired the products of formula (I) are treated with a mineral or organic acid, said products of formula (I) being in

all the possible racemic or enantiomeric isomer forms.

4. Preparation process for the products of formula (I) as defined in claim 1, characterized in that either a product of formula (II'):

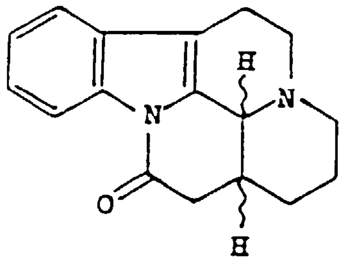

(II')

is subjected

a) **either** to a halogenation reaction in order to obtain the products of formula (III):

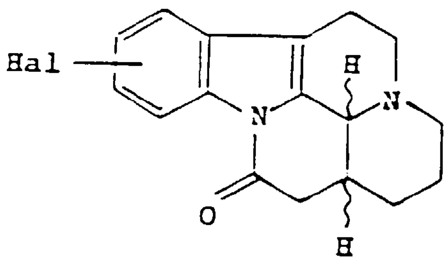

(III)

in which Hal represents a halogen atom, which product of formula (III) is subjected, in any order, to the following reactions:

- a reduction reaction of the oxo function followed by dehydration,
- a substitution reaction of the halogen atom in order to obtain the products of formula ($I'_a$):

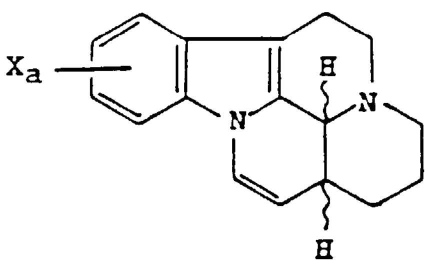

($I'_a$)

in which $X_a$ has the meaning indicated above for $X_1$,

which products of formula ($I'_a$), in the case where $X_a$ represents a formyl radical, can be subjected, if desired, to any one of the conversion reactions of this formyl radical into the carbamoyl, hydroxymethyl or cyano radical, b) **or** to a nitration reaction, in order to obtain the products of formula (IV):

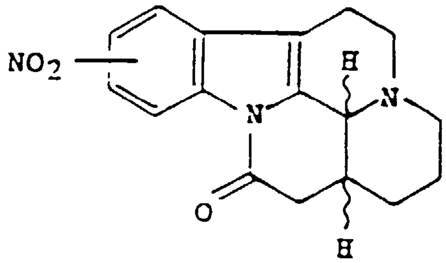

(IV)

which are subjected to a reduction reaction of the nitro radical into the amino radical, in order to obtain the products of formula (V):

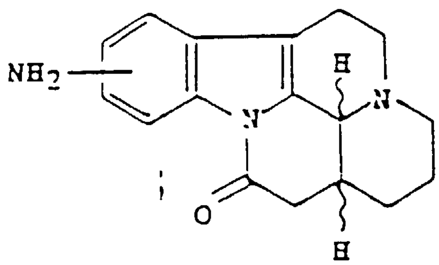

(V)

which product of formula (V) is subjected to the substitution reaction of the amine by a halogen atom via the corresponding diazonium salts in order to obtain the products of formula (III) as defined above,

<u>or</u> a product of formula (II"):

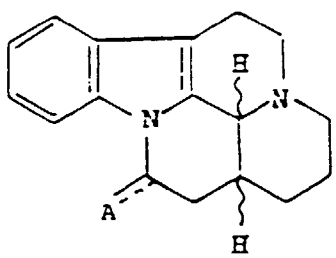

(II")

in which A represents either an oxo function or an

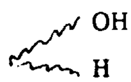

function, is subjected to the action of a compound of formula (IX):

$$X_c - Hal \qquad (IX)$$

in which Hal represents a halogen atom and $X_c$ represents an alkenyl or acyl radical as defined above for $X_1$, in which the reactive functions are optionally protected, in order to obtain the products of formula (X):

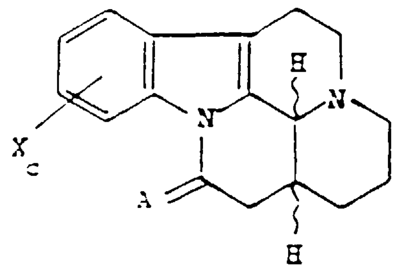

(X)

in which $X_c$ and A have the meaning indicated above, then the products of formula (X) are subjected to a reduction reaction followed by dehydration when =A represents an oxo function or only to a dehydration when A represents an

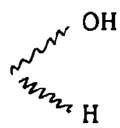

function in order to obtain, after elimination, if necessary and if desired, of the optional protective groups of the reactive functions,
the products of formula (I'$_c$):

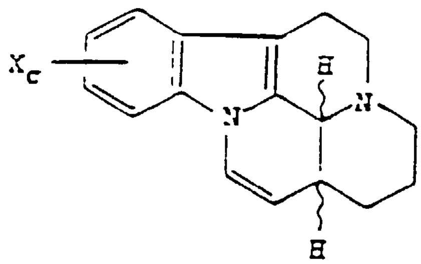

(I'$_c$)

in which $X_c$ has the meaning indicated above, and if desired, the products of formulae (I'$_a$), (I'$_b$), and (I'$_c$) are treated with a mineral or organic acid,
said products of formulae (I'$_a$), (I'$_b$) and (I'$_c$) being in all the possible racemic or enantiomeric isomer forms.

**5.** As medicaments, the products as defined by formula (I) of claim 1, in all the possible racemic or enantiomeric isomer forms, as well the addition salts with pharmaceutically acceptable mineral or organic acids.

**6.** The pharmaceutical compositions containing as active ingredient, at least one of the medicaments as defined in claim 5.

**7.** As new industrial products, the compounds of formula (II"$_B$):

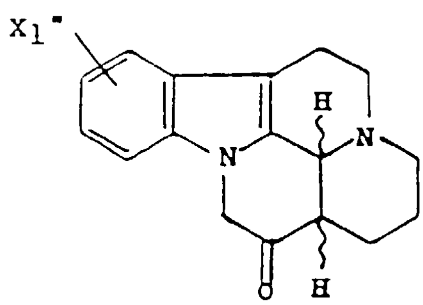

(II"$_B$)

in which $X_1$" is chosen from the group formed by cyano, formyl, acyl or alkoxycarbonyl radicals having 2 to 4 carbon atoms or a carbamoyl radical.

**8.** As new industrial products, the compounds of formula (X).

**Claims for the following Contracting State : ES**

1. Preparation process for the products of formula (I):

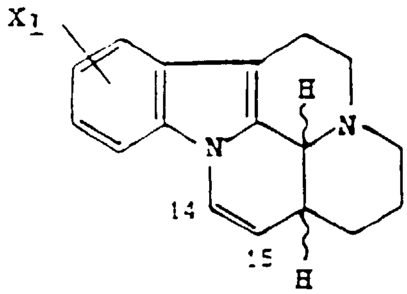

(I)

in which $X_1$ is chosen from the following radicals:

- a linear or branched alkenyl radical containing at most 18 carbon atoms or a hydroxyalkyl radical having at most 8 carbon atoms,
- a cyano, formyl or acyl radical having 2 to 4 carbon atoms,
- an alkoxycarbonyl radical having 2 to 4 carbon atoms or a carbamoyl radical,
- a phenyl radical,

said products of formula (I) being in all the possible racemic or optically active isomer forms, as well as the addition salts with mineral and organic acids of said products of formula (I), characterized in that a product of formula (II):

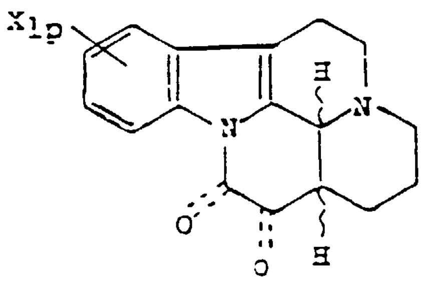

(II)

in which ==O indicates that the single oxo function can be found in position 14 or 15, and $X_{1p}$ represents $X_1$, which has the meaning indicated above, in which the reactive functions are optionally protected, is subjected to a reduction reaction followed by a dehydration and, if necessary, an elimination of the protective groups of the reactive functions which can be carried by $X_{1p}$, in order to obtain the products of formula (I) as defined above, and if desired the products of formula (I) are treated with a mineral or organic acid.

2. Process according to claim 1, characterized in that a product of formula (II) is used at the start in which the oxo function is in position 14.

3. Process according to claim 1, characterized in that a product of formula (II) is used at the start in which the oxo function is in position 15.

4. Process according to claim 1 for the preparation of the products of formula (I) as defined in claim 1, characterized in that <ins>either</ins> a product of formula (II'):

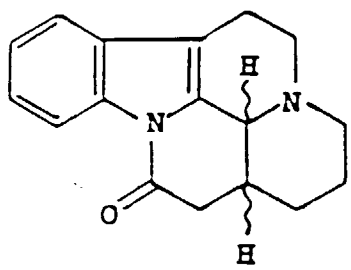

(II')

is subjected

a) **either** to a halogenation reaction
in order to obtain the products of formula (III):

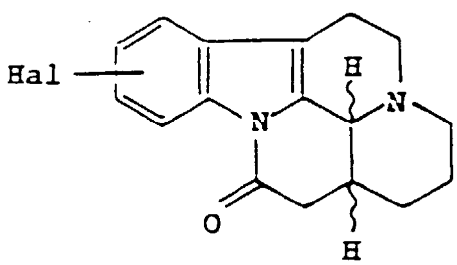

(III)

in which Hal represents a halogen atom, which product of formula (III) is subjected, in any order, to the following reactions:

- a reduction reaction of the oxo function followed by dehydration,
- a substitution reaction of the halogen atom in order to obtain the products of formula ($I'_a$):

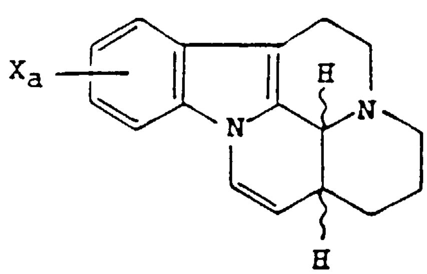

($I'_a$)

in which $X_a$ has the meaning indicated above for $X_1$,
which products of formula ($I'_a$), in the case where $X_a$ represents a formyl radical, can be subjected, if desired, to any one of the conversion reactions of this formyl radical into the carbamoyl, hydroxymethyl or cyano radical,

b) **or** to a nitration reaction,
in order to obtain the products of formula (IV):

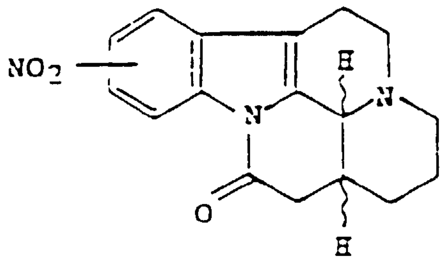

(IV)

which are subjected to a reduction reaction of the nitro radical into the amino radical, in order to obtain the products of formula (V):

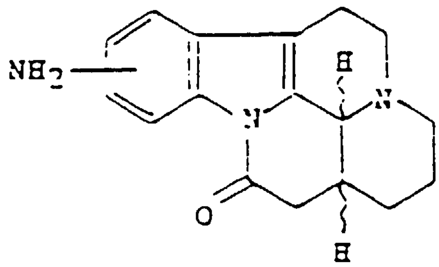

(V)

which product of formula (V) is subjected to the substitution reaction of the amine by a halogen atom via the corresponding diazonium salts in order to obtain the products of formula (III) as defined above,

<u>or</u> a product of formula (II"):

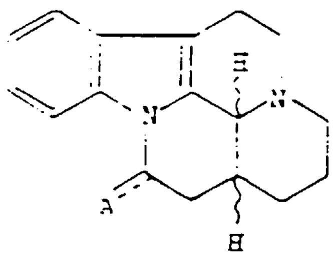

(II")

in which $\overset{-}{=}$ A represents either an oxo function or an

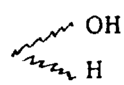

function, is subjected to the action of a compound of formula (IX):

$$X_c - Hal \qquad (IX)$$

in which Hal represents a halogen atom and $X_c$ represents an alkenyl or acyl radical as defined above for $X_1$, in which the reactive functions are optionally protected,
in order to obtain the products of formula (X):

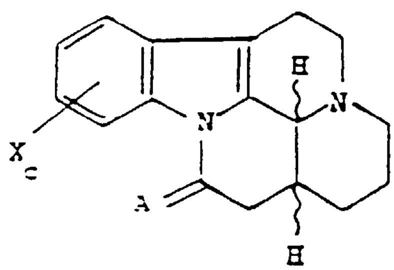

(X)

in which $X_c$ and A have the meaning indicated above, then the products of formula (X) are subjected to a reduction reaction followed by dehydration when =A represents an oxo function or only to a dehydration when A represents an

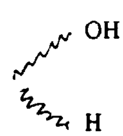

function in order to obtain, after elimination, if necessary and if desired, of the optional protective groups of the reactive functions,
the products of formula ($I'_c$):

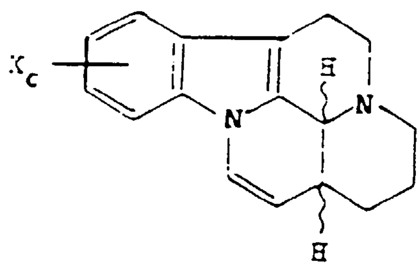

(I'c)

in which $X_c$ has the meaning indicated above, and if desired, the products of formulae (I'a), (I'b), and (I'c) are treated with a mineral or organic acid,
said products of formulae (I'a), (I'b) and (I'c) being in all the possible racemic or enantiomeric isomer forms.

**5.** Process according to claim 1, characterized in that a product of formula (II) is used at the start in which $X_{1p}$ represents $X_1$, in which the reactive functions are optionally protected, $X_1$ being chosen from the following radicals:

- a linear or branched alkenyl radical containing at most 18 carbon atoms or a hydroxyalkyl radical having at most 8 carbon atoms,
- a cyano, formyl or acyl radical having 2 to 4 carbon atoms,
- an alkoxycarbonyl radical having 2 to 4 carbon atoms or a carbamoyl radical,
- a phenyl radical.

**Claims for the following Contracting State : GR**

**1.** Preparation process for the products of formula (I):

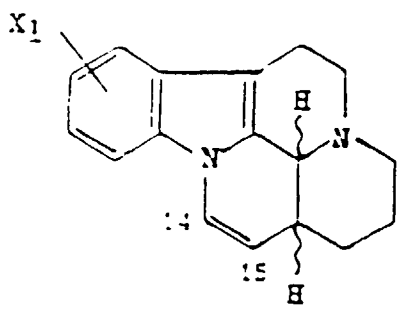

(I)

in which $X_1$ is chosen from the following radicals:

- a linear or branched alkenyl radical containing at most 18 carbon atoms or a hydroxyalkyl radical having at most 8 carbon atoms,
- a cyano, formyl or acyl radical having 2 to 4 carbon atoms,
- an alkoxycarbonyl radical having 2 to 4 carbon atoms or a carbamoyl radical,
- a phenyl radical,

said products of formula (I) being in all the possible racemic or optically active isomer forms, as well as the addition salts with mineral and organic acids of said products of formula (I), characterized in that a product of formula (II):

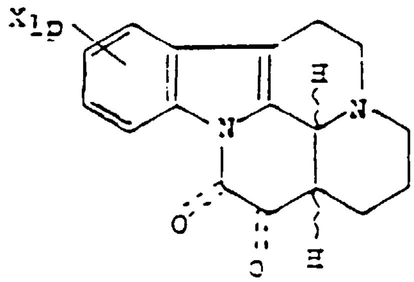

(II)

in which ==O indicates that the single oxo function can be found in position 14 or 15, and $X_{1p}$ represents $X_1$, which has the meaning indicated above, in which the reactive functions are optionally protected, is subjected to a reduction reaction followed by a dehydration and, if necessary, an elimination of the protective groups of the reactive functions which can be carried by $X_{1p}$, in order to obtain the products of formula (I) as defined above, and if desired the products of formula (I) are treated with a mineral or organic acid.

**2.** Process according to claim 1, characterized in that a product of formula (II) is used at the start in which the oxo function is in position 14.

**3.** Process according to claim 1, characterized in that a product of formula (II) is used at the start in which the oxo function is in position 15.

**4.** Process according to claim 1 for the preparation of the products of formula (I) as defined in claim 1, characterized in that either a product of formula (II'):

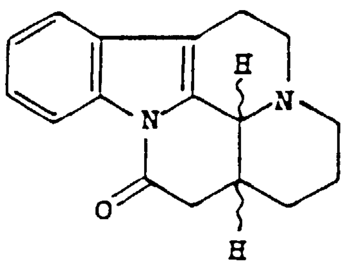

(II')

is subjected

a) **either** to a halogenation reaction
in order to obtain the products of formula (III):

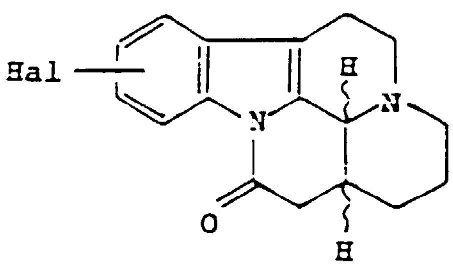

(III)

in which Hal represents a halogen atom, which product of formula (III) is subjected, in any order, to the following reactions:

- a reduction reaction of the oxo function followed by dehydration,
- a substitution reaction of the halogen atom in order to obtain the products of formula ($I'_a$):

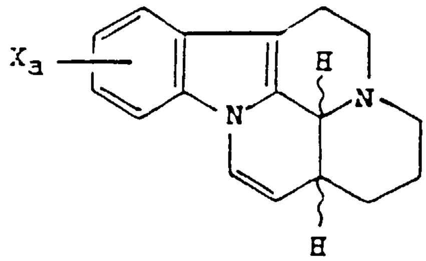

(I'$_a$)

in which $X_a$ has the meaning indicated above for $X_1$, which products of formula (I'$_a$), in the case where $X_a$ represents a formyl radical, can be subjected, if desired, to any one of the conversion reactions of this formyl radical into the carbamoyl, hydroxymethyl or cyano radical,

b) **or** to a nitration reaction, in order to obtain the products of formula (IV):

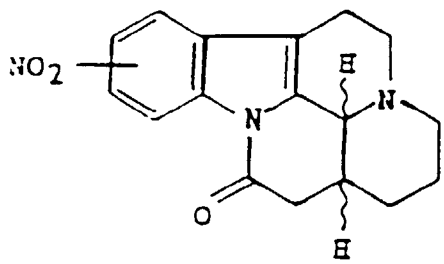

(IV)

which are subjected to a reduction reaction of the nitro radical into the amino radical, in order to obtain the products of formula (V):

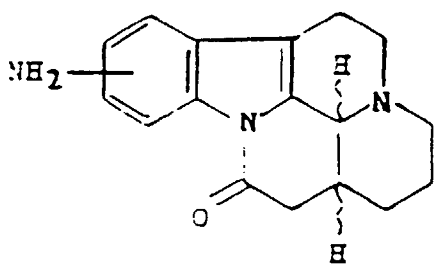

(V)

which product of formula (V) is subjected to the substitution reaction of the amine by a halogen atom via the corresponding diazonium salts in order to obtain the products of formula (III) as defined above,

or a product of formula (II"):

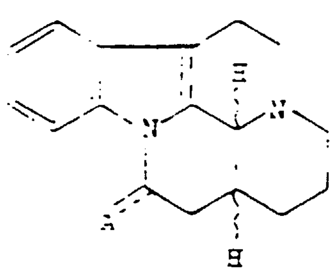

(II")

in which ⩦A represents either an oxo function or an

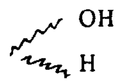

function, is subjected to the action of a compound of formula (IX):

$$X_c - Hal \qquad (IX)$$

in which Hal represents a halogen atom and $X_c$ represents an alkenyl or acyl radical as defined above for $X_1$, in which the reactive functions are optionally protected,
in order to obtain the products of formula (X):

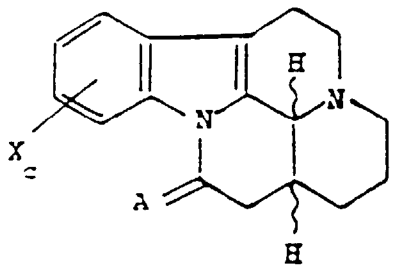

(X)

in which $X_c$ and A have the meaning indicated above, then the products of formula (X) are subjected to a reduction reaction followed by dehydration when =A represents an oxo function or only to a dehydration when A represents an

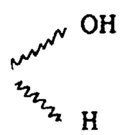

function in order to obtain, after elimination, if necessary and if desired, of the optional protective groups of the reactive functions,
the products of formula ($I'_c$):

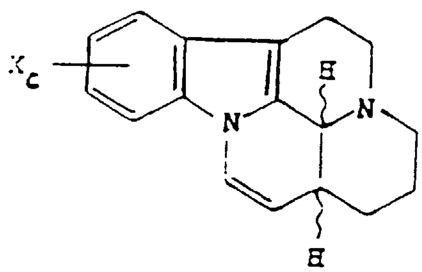

($I'_c$)

in which $X_c$ has the meaning indicated above, and if desired, the products of formulae ($I'_a$), ($I'_b$), and ($I'_c$) are treated with a mineral or organic acid,
said products of formulae ($I'_a$), ($I'_b$) and ($I'_c$) being in all the possible racemic or enantiomeric isomer forms.

**5.** Process according to claim 1, characterized in that a product of formula (II) is used at the start in which $X_{1p}$ represents $X_1$, in which the reactive functions are optionally protected, $X_1$ being chosen from the following radicals:

- a linear or branched alkenyl radical containing at most 18 carbon atoms or a hydroxyalkyl radical having at most 8 carbon atoms,
- a cyano, formyl or acyl radical having 2 to 4 carbon atoms,
- an alkoxycarbonyl radical having 2 to 4 carbon atoms or a carbamoyl radical,
- a phenyl radical.

**6.** Process according to any one of claims 1 to 5,
characterized in that the following are prepared:

- [16alpha (±)]-11-ethenyl-20,21-dinoreburnamenine maleate,
- [16alpha (±)]-11-acetyl-20,21-dinoreburnamenine maleate,
- [16alpha (±)]-alpha-methyl-20,21-dinoreburnamenin-11-methanol fumarate,
- [16alpha (±)]-20,21-dinoreburnamenin-11-carboxamide.

**7.** Preparation process for pharmaceutical compositions, characterized in that at least one of the products of formula (I), as defined in claim 1, in all the possible racemic and enantiomeric isomer forms, or at least one of their addition salts with pharmaceutically acceptable mineral or organic acids is used as active ingredient in a form intended for this use.

**8.** Preparation process for pharmaceutical compositions, characterized in that at least one of the products of formula (I), as defined in claim 6, in all the possible racemic and enantiomeric isomer forms, or at least one of their addition salts with pharmaceutically acceptable mineral or organic acids is used as active ingredient in a form intended for this use.

**9.** As new industrial products, the compounds of formula ($II"_B$):

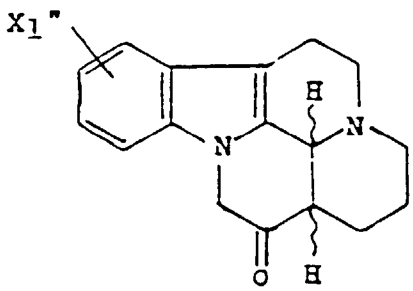

($II"_B$)

in which $X_1$" is chosen from the group formed by cyano, formyl, acyl or alkoxycarbonyl radicals having 2 to 4 carbon atoms or a carbamoyl radical.

**10.** As new industrial products, the compounds of formula (X).

**Patentansprüche**

**Patentansprüche für folgende Vertragsstaaten : AT, BE, CH, DE, DK, FR, GB, IT, LI, LU, NL, SE**

**1.** Produkte der Formel (I):

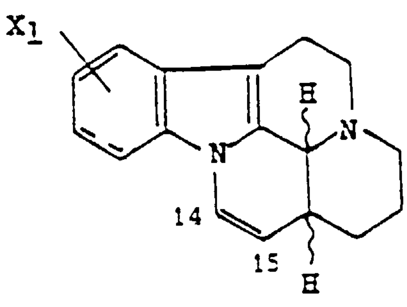

(I)

worin bedeuten:
$X_1$ ist ausgewählt unter den folgenden Resten:

- einem linearen oder verzweigten Alkenylrest mit höchstens 18 Kohlenstoffatomen oder Hydroxyalkyl mit höchstens 8 Kohlenstoffatomen,

- einem Cyano-, Formyl- oder Acylrest mit 2 bis 4 Kohlenstoffatomen,

- einem Alkoxycarbonylrest mit 2 bis 4 Kohlenstoffatomen oder Carbamoyl,

- einem Phenylrest,

wobei diese Produkte der Formel (I) unter allen möglichen isomeren, racemischen, enantiomeren und diastereoisomeren Formen sind, sowie die Additionssalze mit Mineral- und organischen Säuren dieser Produkte der Formel (I).

**2.** Das

- [16alpha (±)]-11-Ethenyl-20,21-dinoreburnamenin-maleat,
- [16alpha (±)]-11-Acetyl-20,21-dinoreburnamenin-maleat,
- [16alpha (±)]-alpha-methyl-20,21-dinoreburnamenin-11-methanol-fumarat,
- [16alpha (±)]-20,21-dinoreburnamenin-11-carboxamid.

**3.** Verfahren zur Herstellung der Produkte der Formel (I), wie in Anspruch definiert, dadurch gekennzeichnet, daß man ein Produkt der Formel (II):

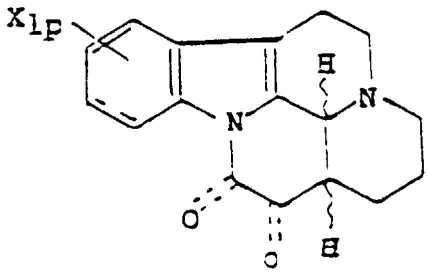

(II)

worin ==O anzeigt, daß die einzige Oxofunktion sich in 14- oder 15-Stellung befinden kann, und $X_{1p}$ $X_1$ bedeutet, welches die in Anspruch 1 angegebene Bedeutung hat, worin die reaktionsfähigen Funktionen gegebenenfalls geschützt sind, einer Reduktionsreaktion unterwirft, gefolgt von einer Dehydratisierung, und falls notwendig, einer Entfernung der Schutzgruppen der reaktiven Funktionen, welche $X_{1p}$ tragen kann, um die Produkte der Formel (I), wie in Anspruch 1 definiert, zu erhalten, und man falls gewünscht die Produkte der Formel (I) mit einer Mineral- oder organischen Säure behandelt, wobei diese Produkte der Formel (I) unter allen möglichen isomeren, racemischen oder enantiomeren Formen vorliegen.

**4.** Verfahren zur Herstellung der Produkte der Formel (I), wie in Anspruch 1 definiert, dadurch gekennzeichnet, daß man
<u>entweder</u> ein Produkt der Formel (II'):

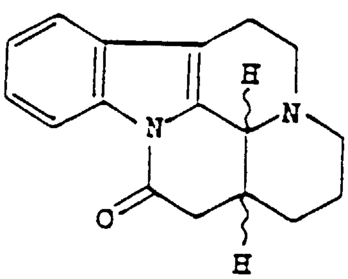

(II')

a) entweder einer Halogenierungsreaktion unterwirft, um die Produkte der Formel (III):

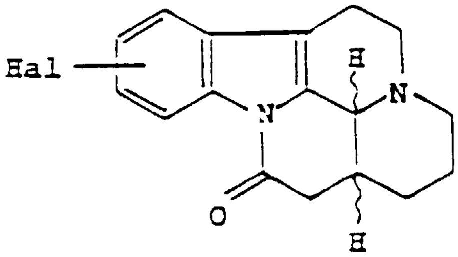

(III)

zu erhalten, worin Hal ein Halogenatom bedeutet, und das Produkt der Formel (III) in irgendeiner Reihenfolge den folgenden Reaktionen unterwirft:

- einer Reduktionsreaktion der Oxofunktion gefolgt von Dehydratisierung,
- einer Substitutionsreaktion des Halogenatoms, um die Produkte der Formel ($I'_a$):

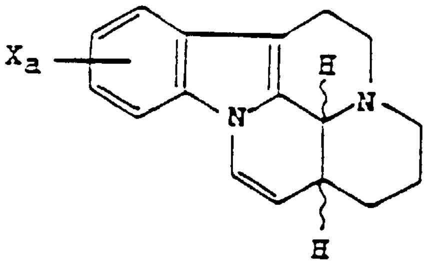

($I'_a$)

zu erhalten, worin $X_a$ die oben für $X_1$ angegebene Bedeutung hat, und man die Produkte der Formel ($I'_a$), falls $X_a$ einen Formylrest bedeutet, gewünschtenfalls irgendeiner der Umwandlungsreaktionen dieses Formylrestes zum Carbamoyl-, Hydroxymethyl- oder Cyanorest unterwerfen kann,

b) oder einer Nitrierungsreaktion unterwirft, um die Produkte der Formel (IV):

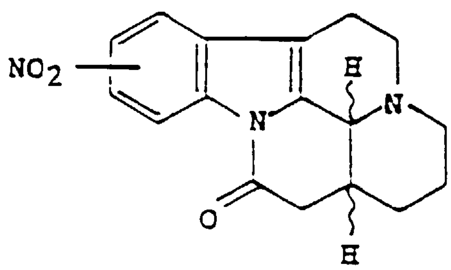

(IV)

zu erhalten, die man einer Reduktionsreaktion des Nitrorestes zum Aminorest unterwirft, um die Produkte der Formel (V):

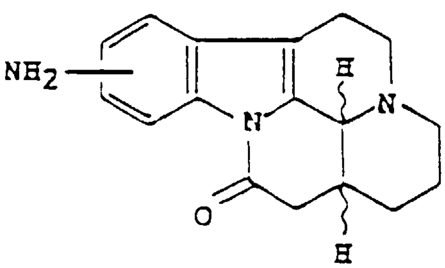

(V)

zu erhalten, wobei man das Produkt der Formel (V) der Substitutionsreaktion des Amins durch ein Halogenatom über die entsprechenden Diazoniumsalze unterwirft, um die Produkte der Formel (III), wie vorstehend definiert, zu erhalten,

<u>oder</u> man unterwirft ein Produkt der Formel (II"):

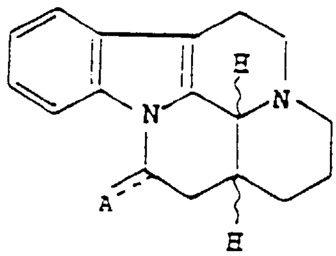

(II")

worin = A entweder eine Oxofunktion oder eine Funktion

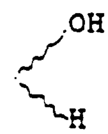

bedeutet,
der Einwirkung einer Verbindung der Formel (IX):

$X_c$ - Hal (IX)

wobei Hal ein Halogenatom darstellt, und $X_c$ einen Alkenyl- oder Acylrest, wie vorstehend für $X_1$ definiert, bedeutet, worin die reaktionsfähigen Funktionen gegebenenfalls geschützt sind,
um Produkte der Formel (X):

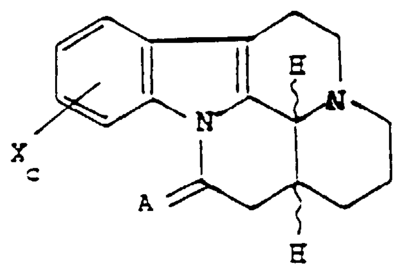

(X)

zu erhalten, worin $X_c$ und A die oben angegebene Bedeutung haben, dann die Produkte der Formel (X) einer Reduktionsreaktion unterwirft, gefolgt von Dehydratisierung, wenn =A eine Oxofunktion bedeutet, oder nur einer Dehydratisierung unterwirft, wenn A eine Funktion

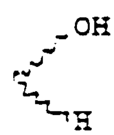

bedeutet, um, falls notwendig und falls erwünscht, nach Entfernung der eventuellen Schutzgruppen der reaktionsfähigen Funktionen die Produkte der Formel (I'$_c$):

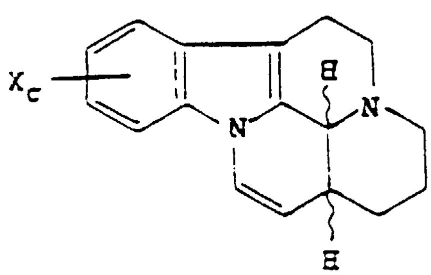

(I'$_c$)

zu erhalten, worin $X_c$ die oben angegebene Bedeutung hat, und, falls gewünscht, die Produkte der Formeln (I'$_a$), (I'$_b$) und (I'$_c$) mit einer Mineral- oder organischen Säure behandelt,

wobei diese Produkte der Formel ($I'_a$), ($I'_b$) und ($I'_c$) unter allen möglichen racemischen oder enantiomeren Formen vorliegen.

**5.** Als Arzneimittel die Produkte, wie sie durch die Formel (I) von Anspruch 1 definiert sind, unter allen möglichen isomeren, racemischen oder enantiomeren Formen, sowie ihre Additionssalze mit pharmazeutisch annehmbaren Mineral- oder organischen Säuren.

**6.** Pharmazeutische Zusammensetzungen, welche als Wirkstoff wenigstens eines der Arzneimittel, wie sie in Anspruch 5 definiert sind, enthalten.

**7.** Als neue industrielle Produkte die Verbindungen der Formel ($II''_B$):

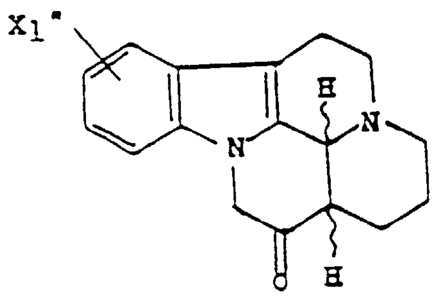

($II''_B$)

worin $X_1$" ausgewählt ist aus der Gruppe gebildet durch die Reste Cyano, Formyl, Acyl oder Alkoxycarbonyl mit 2 bis 4 Kohlenstoffatomen oder Carbamoyl.

**8.** Als neue industrielle Produkte die Verbindungen der Formel (X).

**Patentansprüche für folgenden Vertragsstaat : ES**

**1.** Verfahren zur Herstellung der Produkte der Formel (I):

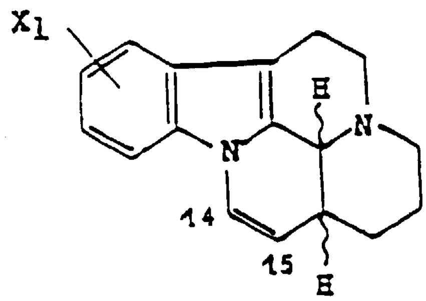

(I)

worin
$X_1$ ausgewählt ist unter den folgenden Resten:

- einem linearen oder verzweigten Alkenylrest mit höchstens 18 Kohlenstoffatomen oder Hydroxyalkyl mit höchstens 8 Kohlenstoffatomen,
- einem Cyano-, Formyl- oder Acylrest mit 2 bis 4 Kohlenstoffatomen,
- einem Alkoxycarbonylrest mit 2 bis 4 Kohlenstoffatomen oder Carbamoyl,
- einem Phenylrest,

wobei diese Produkte der Formel (I) unter allen möglichen isomeren, racemischen oder optisch aktiven Formen vorliegen, sowie der Additionssalze mit den Mineral- und organischen Säuren dieser Produkte der Formel (I),

dadurch gekennzeichnet, daß man ein Produkt der Formel (II):

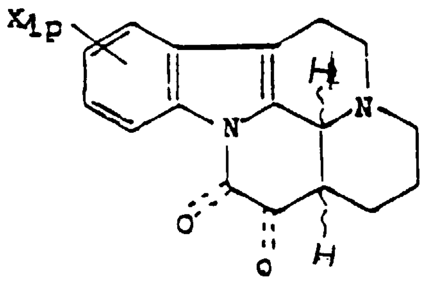

(II)

worin ==O anzeigt, daß die einzige Oxofunktion sich in 14- oder 15-Stellung befinden kann, und $X_{1p}$ $X_1$ bedeutet, welches die oben angegebene Bedeutung hat, worin die reaktionsfähigen Funktionen gegebenenfalls geschützt sind, einer Reduktionsreaktion unterwirft, gefolgt von einer Dehydratisierung, und falls notwendig einer Entfernung der Schutzgruppen der reaktionsfähigen Funktionen, welche $X_{1p}$ tragen kann, um die Produkte der Formel (I ), wie oben definiert, zu erhalten, und man gewünschtenfalls die Produkte der Formel (I) mit einer Mineral- oder organischen Säure behandelt.

**2.** Verfahren gemäß Anspruch 1, dadurch gekennzeichnet, daß man zu Beginn ein Produkt der Formel (II) verwendet, worin die Oxofunktion in 14-Stellung ist.

**3.** Verfahren gemäß Anspruch 1, dadurch gekennzeichnet, daß man zu Beginn ein Produkt der Formel (II) verwendet, worin die Oxofunktion in 15-Stellung ist.

**4.** Verfahren gemäß Anspruch 1 zur Herstellung der Produkte der Formel (I), wie in Anspruch definiert, dadurch gekennzeichnet, daß man
<u>entweder</u> ein Produkt der Formel (II'):

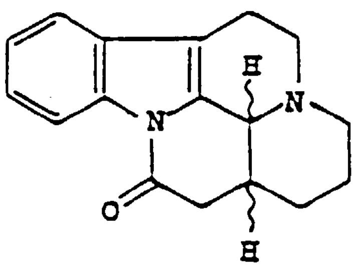

(II')

a) entweder einer Halogenierungsreaktion unterwirft, um die Produkte der Formel (III):

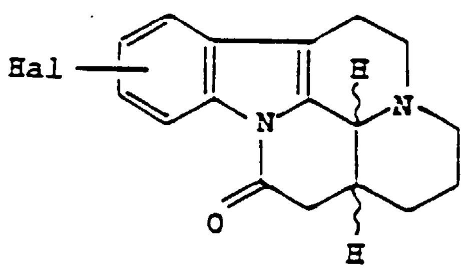

(III)

zu erhalten, worin Hal ein Halogenatom bedeutet, welches Produkt der Formel (III) man in irgendeiner Reihenfolge den folgenden Reaktionen unterwirft:

- einer Reduktionsreaktion der Oxofunktion gefolgt von Dehydratisierung,
- einer Substitutionsreaktion des Halogenatoms, um die Produkte der Formel ($I'_a$):

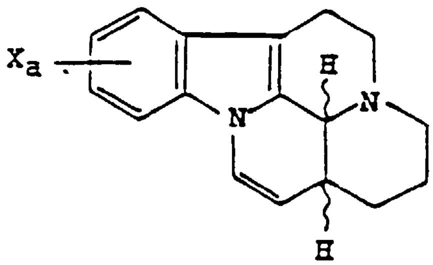

(I'$_a$)

zu erhalten, worin $X_a$ die oben für $X_1$ angegebene Bedeutung hat,
und die Produkte der Formel (I'$_a$) im Fall, daß $X_a$ einen Formylrest bedeutet, man gewünschtenfalls irgendeiner der Umwandlungsreaktionen dieses Formylrestes zum Carbamoyl-, Hydroxymethyl- oder Cyanorest unterwirft,

b) oder einer Nitrierungsreaktion unterwirft, um die Produkte der Formel (IV):

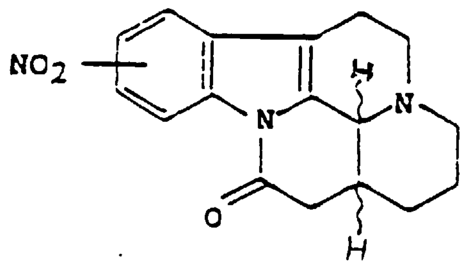

(IV)

zu erhalten, die man einer Reduktionsreaktion des Nitrorestes zum Aminorest unterwirft, um die Produkte der Formel (V):

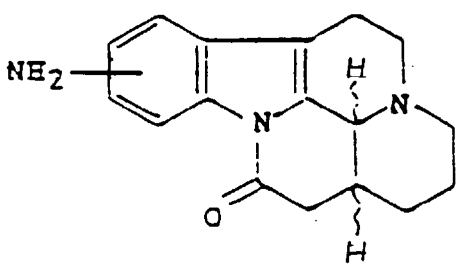

(V)

zu erhalten, welches Produkt der Formel (V) man der Substitutionsreaktion des Amins durch ein Halogenatom über die entsprechenden Diazoniumsalze unterwirft, um die Produkte der Formel (III), wie vorstehend definiert, zu erhalten,

<u>oder</u> man unterwirft ein Produkt der Formel (II"):

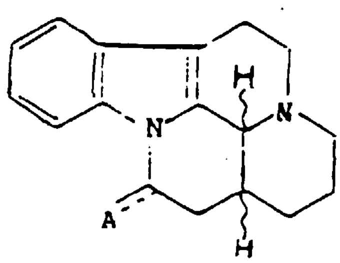

(II")

worin ≂ A entweder eine Oxofunktion oder eine Funktion

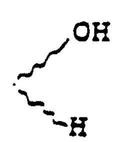

bedeutet,

der Einwirkung einer Verbindung der Formel (IX):

$$X_c \text{ - Hal} \qquad \text{(IX)}$$

wobei Hal ein Halogenatom darstellt, und $X_c$ einen Alkenyl- oder Acylrest, wie vorstehend für $X_1$ definiert, bedeutet, worin die reaktionsfähigen Funktionen gegebenenfalls geschützt sind,
um Produkte der Formel (X):

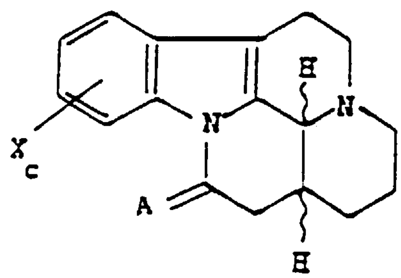

(X)

zu erhalten, worin $X_c$ und A die oben angegebene Bedeutung haben, dann die Produkte der Formel (X) einer Reduktion, gefolgt von einer Dehydratisierung unterwirft, wenn =A eine Oxofunktion bedeutet, oder nur einer Dehydratisierung unterwirft, wenn A eine Funktion

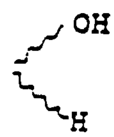

bedeutet, um nach Entfernung, falls notwendig und falls erwünscht, eventueller Schutzgruppen der reaktionsfähigen Funktionen die Produkte der Formel (I'$_c$):

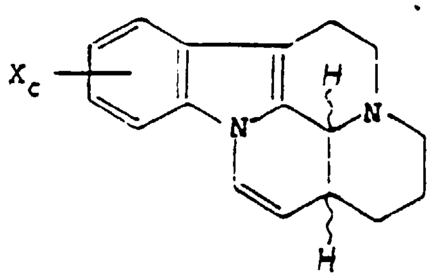

(I'$_c$)

zu erhalten, worin $X_c$ die oben angegebene Bedeutung hat,und man, falls gewünscht, die Produkte der Formeln (I'$_a$), (I'$_b$) und (I'$_c$) mit einer Mineral- oder organischen Säure behandelt,
wobei diese Produkte der Formel (I'$_a$), (I'$_b$) und (I'$_c$) unter allen möglichen isomeren, racemischen oder enantiomeren Formen vorliegen.

**5.** Verfahren gemäß Anspruch 1, dadurch gekennzeichnet, daß man zu Beginn ein Produkt der Formel (II) verwendet, worin $X_{1p}$ $X_1$ bedeutet, worin die reaktionsfähigen Funktionen gegebenenfalls geschützt sind, wobei $X_1$ ausgewählt ist unter den folgenden Resten:

- einem linearen oder verzweigten Alkenylrest mit höchstens 18 Kohlenstoffatomen oder Hydroxyalkyl mit höchstens 8 Kohlenstoffatomen,
- einem Cyano-, Formyl- oder Acylrest mit 2 bis 4 Kohlenstoffatomen,
- einem Alkoxycarbonylrest mit 2 bis 4 Kohlenstoffatomen oder Carbamoyl,
- einem Phenylrest.

**Patentansprüche für folgenden Vertragsstaat : GR**

1. Verfahren zur Herstellung von Produkten der Formel (I):

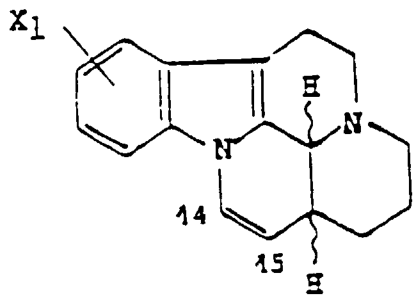

(I)

worin
$X_1$ ausgewählt ist unter den folgenden Resten:

- einem linearen oder verzweigten Alkenylrest mit höchstens 18 Kohlenstoffatomen oder Hydroxyalkyl mit höchstens 8 Kohlenstoffatomen,
- einem Cyano-, Formyl- oder Acylrest mit 2 bis 4 Kohlenstoffatomen,
- einem Alkoxycarbonylrest mit 2 bis 4 Kohlenstoffatomen oder Carbamoyl,
- einem Phenylrest,

wobei diese Produkte der Formel (I) unter allen möglichen isomeren, racemischen oder optisch aktiven Formen vorliegen, sowie den Additionssalzenmit den Mineral- und organischen Säuren dieser Produkte der Formel (I), dadurch gekennzeichnet, daß man ein Produkt der Formel (II):

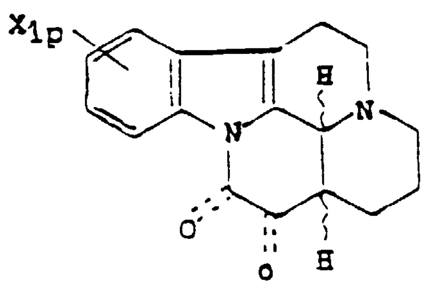

(II)

worin ==O anzeigt, daß die einzige Oxofunktion sich in 14- oder 15-Stellung befinden kann, und $X_{1p}$ $X_1$ bedeutet, welches die oben angegebene Bedeutung hat, worin die reaktionsfähigen Funktionen gegebenenfalls geschützt sind, einer Reduktionsreaktion unterwirft, gefolgt von einer Dehydratisierung, und falls notwendig einer Entfernung der Schutzgruppen der reaktionsfähigen Funktionen, welche $X_{1p}$ tragen kann, um die Produkte der Formel (I), wie oben definiert, zu erhalten, und man gewünschtenfalls die Produkte der Formel (I) mit einer Mineral- oder organischen Säure behandelt.

2. Verfahren gemäß Anspruch 1, dadurch gekennzeichnet, daß man zu Beginn ein Produkt der Formel (II) verwendet, worin die Oxofunktion in 14-Stellung ist.

3. Verfahren gemäß Anspruch 1, dadurch gekennzeichnet, daß man zu Beginn ein Produkt der Formel (II) verwendet, worin die Oxofunktion in 15-Stellung ist.

4. Verfahren gemäß Anspruch 1 zur Herstellung der Produkte der Formel (I), wie in Anspruch 1 definiert, dadurch gekennzeichnet, daß man
<u>entweder</u> ein Produkt der Formel (II'):

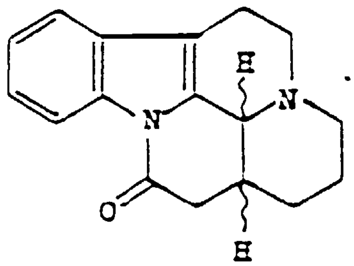

(II')

unterwirft

a) entweder einer Halogenierungsreaktion, um die Produkte der Formel (III):

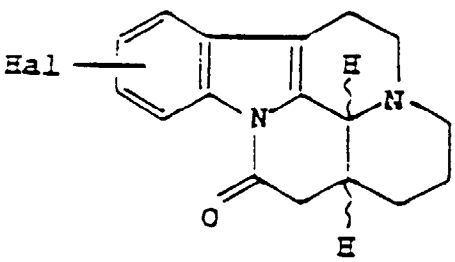

(III)

zu erhalten, worin Hal ein Halogenatom bedeutet, man das Produkt der Formel (III) in irgendeiner Reihenfolge den folgenden Reaktionen unterwirft:

- einer Reduktionsreaktion der Oxofunktion gefolgt von Dehydratisierung,
- einer Substitutionsreaktion des Halogenatoms, um die Produkte der Formel ($I'_a$):

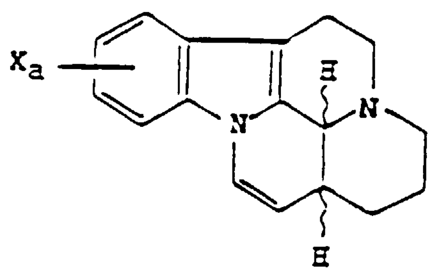

($I'_a$)

zu erhalten, worin $X_a$ die oben für $X_1$ angegebene Bedeutung hat,
und die Produkte der Formel ($I'_a$) im Fall, daß $X_a$ einen Formylrest bedeutet, man gewünschtenfalls irgendeiner der Umwandlungsreaktionen dieses Formylrestes zum Carbamoyl-, Hydroxymethyl- oder Cyanorest unterwerfen kann,

b) oder einer Nitrierungsreaktion, um die Produkte der Formel (IV):

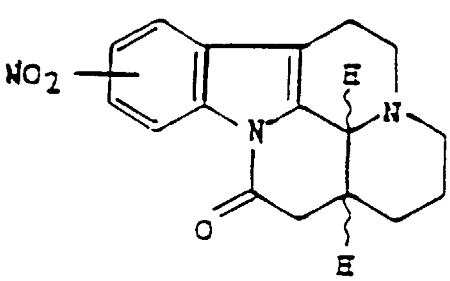

(IV)

zu erhalten, die man einer Reduktionsreaktion des Nitrorestes zum Aminorest unterwirft,
um die Produkte der Formel (V):

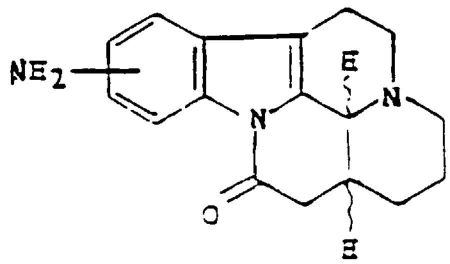

(V)

zu erhalten, welches Produkt der Formel (V) man der Substitutionsreaktion des Amins durch ein Halogenatom über die entsprechenden Diazoniumsalze unterwirft, um die Produkte der Formel (III), wie vorstehend definiert, zu erhalten,

oder man unterwirft ein Produkt der Formel (II"):

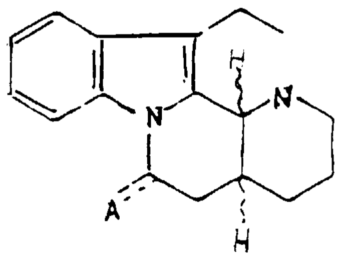

(II")

worin = A entweder eine Oxofunktion oder eine Funktion

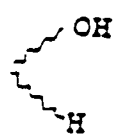

bedeutet,
der Einwirkung einer Verbindung der Formel (IX):

$$X_c \text{ - Hal} \qquad \text{(IX)}$$

worin Hal ein Halogenatom darstellt, und $X_c$ einen Alkenyl- oder Acylrest, wie vorstehend für $X_1$ definiert, bedeutet, worin die reaktionsfähigen Funktionen gegebenenfalls geschützt sind,
um Produkte der Formel (X):

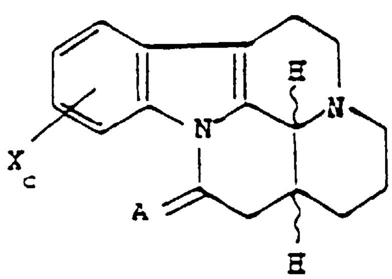

(X)

zu erhalten, worin $X_c$ und A die oben angegebene Bedeutung haben, dann die Produkte der Formel (X) einer Reduktion, gefolgt von einer Dehydratisierung unterwirft, wenn =A eine Oxofunktion bedeutet, oder nur einer Dehydratisierung unterwirft, wenn A eine Funktion

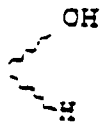

bedeutet, um nach Entfernung, falls notwendig und gewünscht, eventueller Schutzgruppen der reaktionsfähigen Funktionen die Produkte der Formel ($I'_c$):

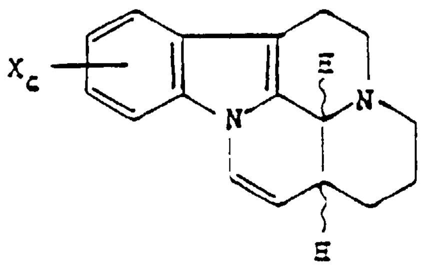

($I'_c$)

zu erhalten, worin $X_c$ die oben angegebene Bedeutung hat,und man, falls gewünscht, die Produkte der Formeln ($I'_a$), ($I'_b$) und ($I'_c$) mit einer Mineral- oder organischen Säure behandelt,
wobei diese Produkte der Formel ($I'_a$), ($I'_b$) und ($I'_c$) unter allen möglichen isomeren, racemischen oder enantiomeren Formen vorliegen.

**5.** Verfahren gemäß Anspruch 1, dadurch gekennzeichnet, daß man zu Beginn ein Produkt der Formel (II) verwendet, worin $X_{1p}$ $X_1$ bedeutet, worin die reaktionsfähigen Funktionen gegebenenfalls geschützt sind, wobei $X_1$ ausgewählt ist unter den folgenden Resten:

- einem linearen oder verzweigten Alkenylrest mit höchstens 18 Kohlenstoffatomen oder Hydroxyalkyl mit höchstens 8 Kohlenstoffatomen,
- einem Cyano-, Formyl- oder Acylrest mit 2 bis 4 Kohlenstoffatomen,
- einem Alkoxycarbonylrest mit 2 bis 4 Kohlenstoffatomen oder Carbamoyl,
- einem Phenylrest.

**6.** Verfahren gemäß einem der Ansprüche 1 bis 5, dadurch gekennzeichnet, daß man herstellt:

- [16alpha (±)]-11-Ethenyl-20,21-dinoreburnamenin-maleat,
- [16alpha (±)]-11-Acetyl-20,21-dinoreburnamenin-maleat,
- [16alpha (±)]-alpha-methyl-20,21-dinoreburnamenin-11-methanol-fumarat,
- [16alpha (±)]-20,21-dinoreburnamenin-11-carboxamid.

**7.** Verfahren zur Herstellung von pharmazeutischen Zusammensetzungen, dadurch gekennzeichnet, daß man als Wirkstoff mindestens eines der Produkte der Formel (I), wie in Anspruch 1 definiert, unter allen möglichen isomeren, racemischen und enantiomeren Formen oder mindestens eines seiner Additionssalze mit pharmazeutisch annehmbaren Mineral- oder organischen Säuren in einer für diese Verwendung bestimmten Form einsetzt.

**8.** Verfahren zur Herstellung von pharmazeutischen Zusammensetzungen, dadurch gekennzeichnet, daß man als Wirkstoff mindestens eines der Produkte der Formel (I), wie in Anspruch 6 definiert, unter allen möglichen isomeren, racemischen und enantiomeren Formen oder mindestens eines seiner Additionssalze mit pharmazeutisch annehmbaren Mineral- oder organischen Säuren in einer für diese Verwendung bestimmten Form einsetzt.

**9.** Als neue industrielle Produkte die Verbindungen der Formel ($II''_B$) :

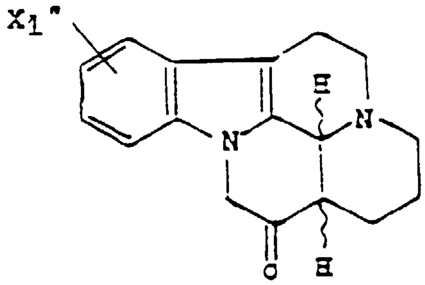

(II"B)

worin $X_1$" ausgewählt ist aus der Gruppe gebildet durch die Reste Cyano, Formyl, Acyl oder Alkoxycarbonyl mit 2 bis 4 Kohlenstoffatomen oder Carbamoyl.

**10.** Als neue industrielle Produkte die Verbindungen der Formel (X).